(19) Europäisches Patentamt
European Patent Office
Office européen des brevets

(11) **EP 3 698 792 A1**

(12) **EUROPEAN PATENT APPLICATION**

(43) Date of publication:
26.08.2020 Bulletin 2020/35

(21) Application number: 20160164.8

(22) Date of filing: 08.03.2012

(51) Int Cl.:
*A61K 31/445* (2006.01)     *A61K 38/47* (2006.01)
*A61P 3/00* (2006.01)      *A61K 9/48* (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 11.03.2011  US 201161451798 P
20.12.2011  US 201161578201 P
07.02.2012  US 201261596165 P

(62) Document number(s) of the earlier application(s) in
accordance with Art. 76 EPC:
**12758017.3 / 2 683 382**

(71) Applicant: **Amicus Therapeutics, Inc.**
**Cranbury, NJ 08512 (US)**

(72) Inventors:
• **GREENE, Douglas Stuart**
**Newton, PA Pennsylvania 18940 (US)**
• **VALENZANO, Kenneth Joseph**
**East Brunswick, NJ New Jersey 08816 (US)**

(74) Representative: **Miller Sturt Kenyon**
**9 John Street**
**London WC1N 2ES (GB)**

Remarks:
This application was filed on 28-02-2020 as a
divisional application to the application mentioned
under INID code 62.

(54) **DOSING REGIMENS FOR THE TREATMENT OF FABRY DISEASE**

(57) A pharmaceutical composition comprising migalastat hydrochloride, magnesium stearate and pregelatinized starch.

EP 3 698 792 A1

**Description**

**CROSS-REFERENCE TO RELATED APPLICATIONS**

This application claims priority to U.S. Provisional Application Serial Nos. 61/451,798 filed March 11, 2011; 61/578,201 filed December 20, 2011 and 61/596,165 filed February 7, 2012, the disclosures of which are hereby incorporated by reference in their entireties.

**FIELD OF THE APPLICATION**

The present application provides a dosing regimen and administration schedule for the use of 1-deoxygalactonojirimycin and enzyme replacement therapy for the treatment of Fabry disease.

**BACKGROUND**

Fabry disease is a progressive, X-linked inborn error of glycospingolipid metabolism caused by a deficiency in the lysosomal enzyme $\alpha$-galactosidase A (a-Gal A) as a result of mutations in the $\alpha$-Gal A gene (GLA). Despite being an X-linked disorder, females can express varying degrees of clinical manifestations. Fabry is a rare disease with incidence estimated between 1 in 40,000 males to 1 in 117,000 in the general population. Moreover, there are variants of later-onset phenotype of Fabry disease that can be under-diagnosed, as they do not present with classical signs and symptoms. This, and the study of newborn screening for Fabry disease, suggests that the actual incidence of Fabry disease can be higher than currently estimated.

Clinical manifestation of the disease can correlate with residual $\alpha$-Gal A levels. Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. Gradual deterioration of renal function and the development of azotemia, due to glycospingolipid deposition, usually occur in the third to fifth decades of life, but can occur as early as in the second decade. Renal lesions are found in both hemizygous (male) and heterozygous (female) patients.

Cardiac disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

The current approved treatment for Fabry disease is enzyme replacement therapy ("ERT"). Two $\alpha$-Gal A products are currently available for the treatment of Fabry disease: agalsidase alfa (Replagal®, Shire Human Genetic Therapies) and agalsidase beta (Fabrazyme®; Genzyme Corporation). These two forms of ERT are intended to compensate for a patient's inadequate $\alpha$-Gal A activity with a recombinant form of the enzyme, administered intravenously. While ERT is effective in many settings, the treatment also has limitations. ERT has not been demonstrated to decrease the risk of stroke, cardiac muscle responds slowly, and GL-3 elimination from some of the cell types of the kidneys is limited. Some patients develop immune reactions to ERT.

1-deoxygalactonojirimycin and its salt, 1-deoxygalactonojirimycin hydrochloride (also known by its United States Adopted Name (USAN), migalastat hydrochloride) acts as a pharmacological chaperone for mutant $\alpha$-Gal A by selectively binding to the enzyme, thereby increasing its stability and helping the enzyme fold into its correct three-dimensional shape. This stabilization of $\alpha$-Gal A allows the cell's quality control mechanisms to recognize the enzyme as properly folded so that trafficking of the enzyme to the lysosome is increased, allowing it to carry out its intended biological function, the metabolism of GL-3. As a result of restoring the proper trafficking of $\alpha$-Gal A from the ER to the lysosome, migalastat hydrochloride also reduces the accumulation of misfolded protein in the ER, which can alleviate stress on cells and some inflammatory-like responses that can be contributing factors in Fabry disease. Multiple in vitro and in vivo preclinical studies, as well as clinical studies, of migalastat hydrochloride have been conducted. Migalastat hydrochloride has been shown to increase the amount of intracellular $\alpha$-Gal A protein and to enhance transport of mutant enzyme to the lysosome.

**SUMMARY**

The present application provides a dosing regimen and administration schedule for the use of 1-deoxygalactonojirimycin and enzyme replacement therapy for the treatment of Fabry disease. In certain embodiments, the present application provides a dosing regimen and administration schedule for the use of migalastat hydrochloride and agalsidase (e.g., agalsidase alfa or agalsidase beta) for the treatment of Fabry disease.

In one embodiment, the method includes administering from about 50 mg to about 600 mg of 1-deoxygalactonojirimycin and an effective amount of α-Gal A enzyme replacement therapy to a patient in need thereof. The 1-deoxygalactonojirimycin may be administered before, after, or simultaneously with the α-Gal A enzyme replacement therapy. In one embodiment, the patient fasts for a period of time beginning about 0.5 to about 4 hours prior to and ending about 0.5 to about 4 hours following administration of 1-deoxygalactonojirimycin. In a further embodiment, the patient fasts for at least about 2 hours prior to and at least about 2 hours following administration of 1-deoxygalactonojirimycin.

In another embodiment, the 1-deoxygalactonojirimycin is administered between simultaneously with to about 4 hours prior to the administration of the α-Gal A enzyme replacement therapy (from T=-4 hours to T=0 hours). In a further embodiment, the 1-deoxygalactonojirimycin is administered about 2 hours prior to the administration of the α-Gal A enzyme replacement therapy.

In a particular embodiment, the 1-deoxygalactonojirimycin is migalastat hydrochloride. In one embodiment, the α-Gal A enzyme replacement therapy is agalsidase alfa or agalsidase beta.

In one embodiment, the 1-deoxygalactonojirimycin is administered as an adjuvant to the α-Gal A enzyme replacement therapy. In another embodiment, the 1-deoxygalactonojirimycin and α-Gal A enzyme replacement therapy are administered as a combination therapy.

In a particular embodiment, the amount of 1-deoxygalactonojirimycin administered according to the above-described method is from about 150 mg to about 450 mg. In one embodiment, the amount of 1-deoxygalactonojirimycin administered is selected from 150 mg, 300 mg and 450 mg.

In a particular embodiment, the 1-deoxygalactonojirimycin is administered immediately before or at the same time as the administration of the α-Gal A enzyme replacement therapy. In an alternate embodiment, a second dose of 1-deoxygalactonojirimycin is administered between the administration of the α-Gal A enzyme replacement therapy and 4 hours thereafter.

In certain embodiments, the 1-deoxygalactonojirimycin is administered every 1 to 4 weeks to a patient who is also receiving α-Gal A enzyme replacement therapy. In a further embodiment, the 1-deoxygalactonojirimycin is administered every 12 to 16 days to a patient who is also receiving α-Gal A enzyme replacement therapy. In a further embodiment, the 1-deoxygalactonojirimycin is administered every 14 days to a patient who is also receiving α-Gal A enzyme replacement therapy. In certain embodiments, the α-Gal A enzyme replacement therapy is administered every 14 days to the patient who is also administered 1-deoxygalactonojirimycin as a combination or adjuvant therapy.

The present application also provides 1-deoxygalactonojirimycin for use in the treatment of Fabry disease, wherein the treatment comprises administering from about 50 mg to about 600 mg of 1-deoxygalactonojirimycin and an effective amount of α-Gal A enzyme replacement therapy to a human subject in need thereof.

The present application also provides the use of 1-deoxygalactonojirimycin in the preparation of a medicament for the treatment of Fabry disease, wherein the treatment comprises administering from about 50 mg to about 600 mg of 1-deoxygalactonojirimycin and an effective amount of α-Gal A enzyme replacement therapy to a human subject in need thereof.

The present application also provides a kit for treating Fabry disease in a subject, the kit comprising from about 50 mg to about 600 mg of 1-deoxygalactonojirimycin and an effective amount of α-Gal A enzyme replacement therapy. In certain embodiments, the amount of 1-deoxygalactonojirimycin in the kit is selected from 150 mg, 300 mg and about 450 mg.

## BRIEF DESCRIPTION OF THE DRAWINGS

**Figure** 1 shows plasma α-Gal A activity composites for patients treated during Periods 1 and 2 with 0.5 mg/kg or 1.0 mg/kg agalsidase beta alone (Period 1) or in combination with 150 mg migalastat (Period 2).

**Figure 2** shows plasma α-Gal A activity AUC increases for all patients following Co-administration with migalastat.

**Figure 3** shows the partial AUC's for each sampling time which showed increased activity of plasma α-Gal A activity with co-administration of 0.5 mg/kg or 1.0 mg/kg agalsidase beta and 150 mg migalastat.

**Figure 4A**-B shows the increase in skin α-Gal A activity in two patients following co-administration of 0.5 mg/kg agalsidase beta and 150 mg migalastat.

**Figure 5A**-B shows the increase in skin α-Gal A activity in two patients following co-administration of 0.5 mg/kg agalsidase beta and 150 mg migalastat. Figure 5A shows the increase in skin α-Gal A activity following co-administration of 0.5 mg/kg agalsidase beta and 150 mg migalastat in the patient who received a 40 min. longer ERT infusion during Period 2.

**Figure 6A**-B shows the increase in skin $\alpha$-Gal A activity in two patients following co-administration of 1.0 mg/kg agalsidase beta and 150 mg migalastat.

**Figure 7A**-B shows the increase in PBMC $\alpha$-Gal A activity in two patients following co-administration of 0.5 mg/kg agalsidase beta and 150 mg migalastat.

**Figure 8A**-B shows the increase in PBMC $\alpha$-Gal A activity in two patients following co-administration of 0.5 mg/kg agalsidase beta and 150 mg migalastat.

**Figure 9A**-B shows the increase in PBMC $\alpha$-Gal A activity in two patients following co-administration of 1.0 mg/kg agalsidase beta and 150 mg migalastat.

**Figure 10** shows a table summarizing the increases in $\alpha$-Gal A activity in plasma, skin and PBMC following co-administration of 0.5 mg/kg or 1.0 mg/kg agalsidase beta and 150 mg migalastat.

**Figure 11** shows the genotype for each study subject of Examples 2 and 3.

**Figure 12** shows plasma AUC $\alpha$-Gal A activity versus treatment with 1.0 mg/kg agalsidase beta, and treatment with a combination of 1.0 mg/kg agalsidase beta and 150 mg migalastat HCl (with inserted means and standard deviations).

**Figure 13** shows plasma AUC $\alpha$-Gal A activity versus treatment with 0.5 mg/kg agalsidase beta, and treatment with a combination of 0.5 mg/kg agalsidase beta and 150 mg migalastat HCl (with inserted means and standard deviations).

**Figure 14** shows skin $\alpha$-GAL A activity on Day 2 after treatment with agalsidase beta alone or in combination with 150 mg migalastat HCl (with baseline-subtracted ratios from agalsidase beta alone).

**Figure 15** Skin $\alpha$-GAL A activity on Day 7 after treatment with agalsidase beta alone or in combination with 150 mg migalastat HCl (with baseline-subtracted ratios from agalsidase beta alone).

## DETAILED DESCRIPTION

The present application provides a dosing regimen and administration schedule for the use of 1-deoxygalactonojirimycin and agalsidase for the treatment of Fabry disease.

## Definitions

"Fabry disease" refers to classical Fabry disease, late-onset Fabry disease, and hemizygous females having mutations in the gene encoding $\alpha$-galactosidase A ($\alpha$-Gal A). The term "Fabry disease," as used herein, further includes any condition in which a subject exhibits lower than normal endogenous $\alpha$-Gal A activity.

The term "AUC" represents a mathematical calculation to evaluate the body's total exposure over time to a given drug. In a graph plotting how concentration in the blood after dosing, the drug concentration variable lies on the y-axis and time lies on the x-axis. The area between a drug concentration curve and the x-axis for a designated time interval is the AUC. AUCs are used as a guide for dosing schedules and to compare different drugs' availability in the body.

The term "Cmax" represents the maximum plasma concentration achieved after dosing.

The terms "therapeutically effective dose" and "effective amount" refer to the amount of the specific pharmaceutical compound or composition that is sufficient to result in a beneficial therapeutic response. A beneficial therapeutic response can be any response that a user (*e.g.*, a clinician) will recognize as an effective response to the therapy, including the foregoing symptoms and surrogate clinical markers. Thus, a therapeutic response will generally be an amelioration of one or more symptoms of a disease or disorder, *e.g.*, Fabry disease, such as those known in the art for the disease or disorder, *e.g.*, for Fabry disease.

Non-limiting examples of improvements in surrogate markers for Fabry disease include increases in $\alpha$-GAL levels or activity in cells (*e.g.*, fibroblasts) and tissue; reductions in of GL-3 accumulation as measured by the change in kidney interstitial capillary biopsies using histology; decreased urine GL-3 levels; assessment of renal function (including glomerular filtration rate (GFR) and 24-hour urine protein; decreased plasma concentrations of homocysteine and vascular cell adhesion molecule-1 (VCAM-1); decreased GL-3 accumulation within myocardial cells and valvular fibrocytes; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin (Fuller et al., Clinical Chemistry. 2005; 51: 688-694); the absence of laminated inclusion bodies (Zebra bodies) in glomerular epithelial cells; improvements in renal function; mitigation of hypohidrosis; the absence of angiokeratomas; and improvements hearing abnormalities such as high frequency sensorineural hearing loss progressive hearing loss, sudden deafness, or tinnitus. Improvements in neurological symptoms include prevention of transient ischemic attack (TIA) or stroke; and amelioration of neuropathic pain manifesting itself as acroparaesthesia (burning or tingling in extremities).

The phrase "pharmaceutically acceptable" refers to molecular entities and compositions that are physiologically tolerable and do not typically produce untoward reactions when administered to a human. Preferably, as used herein, the term "pharmaceutically acceptable" means approved by a regulatory agency of the federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 18th Edition, or other editions, which is hereby incorporated by reference in its entirety.

"l-deoxygalactonojirimycin" (DGJ) refers to (2R,3S,4R,5S)-2-(hydroxymethyl) piperdine-3,4,5-triol. As used herein, reference to "1-deoxygalactonojirimycin" or "DGJ" throughout includes both the free base and any pharmaceutically acceptable salt forms of the same. The hydrochloride salt of DGJ is known as migalastat hydrochloride.

The term "adjuvant" or "adjuvant therapy" refers to any additional substance, treatment, or procedure used for increasing the efficacy, safety, or otherwise facilitating or enhancing the performance of a primary substance, treatment, or procedure.

The term "combination therapy" refers to any therapy wherein the results are enhanced as compared to the effect of each therapy when it is performed individually. The individual therapies in a combination therapy may be administered concurrently or consecutively.

Enhancement may include any improvement of the effect of the various therapies that may result in an advantageous result as compared to the results achieved by the therapies when performed alone. Enhanced effect and determination of enhanced effect may be measured by various parameters such as, but not limited to: temporal parameters (*e.g.*, length of treatment, recovery time, long-term effect of the treatment or reversibility of treatment); biological parameters (*e.g.*, cell number, cell volume, cell composition, tissue volume, tissue size, tissue composition); spatial parameters (*e.g.*, tissue strength, tissue size or tissue accessibility) and physiological parameters (*e.g.*, body contouring, pain, discomfort, recovery time or visible marks). Enhanced effect may include a synergistic enhancement, wherein the enhanced effect is more than the additive effects of each therapy when performed by itself. Enhanced effect may include an additive enhancement, wherein the enhanced effect is substantially equal to the additive effect of each therapy when performed by itself. Enhanced effect may include less than a synergistic effect, wherein the enhanced effect is lower than the additive effect of each therapy when performed by itself, but still better than the effect of each therapy when performed by itself.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" can mean values that are within an order of magnitude, preferably within 5-fold and more preferably within 2-fold of a given value. Numerical quantities given herein are approximate unless stated otherwise.

**Formulation and Administration**

1-deoxygalactonojirimycin can be administered as the free base or as a pharmacologically acceptable salt form, including 1-deoxygalactonojirimycin hydrochloride (a.k.a., migalastat hydrochloride). It can be administered in a form suitable for any route of administration, including e.g., orally in the form tablets, capsules, or liquid, or in sterile aqueous solution for injection. It can be administered orally in the form of tablets, capsules, ovules, elixirs, solutions or suspensions, gels, syrups, mouth washes, or a dry powder for constitution with water or other suitable vehicle before use, optionally with flavoring and coloring agents for immediate-, delayed-, modified-, sustained-, pulsed-or controlled-release applications. Solid compositions such as tablets, capsules, lozenges, pastilles, pills, boluses, powder, pastes, granules, bullets, or premix preparations can also be used. Solid and liquid compositions for oral use can be prepared according to methods well known in the art. Such compositions can also contain one or more pharmaceutically acceptable carriers and excipients which can be in solid or liquid form. When the compound is formulated for oral administration, the tablets or capsules can be prepared by conventional means with pharmaceutically acceptable excipients such as binding agents (e.g., pregelatinized starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets can be coated by methods well known in the art.

The pharmaceutically acceptable excipients also include, but are not limited to, microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrolidone, hydroxypropyl ethylcellulose (HPMC), hydroxypropyl cellulose (HPC), sucrose, gelatin, and acacia. Additionally, lubricating agents such as magnesium stearate, stearic acid, glyceryl behenate and talc can be included.

In a specific embodiment, migalastat hydrochloride is formulated with magnesium stearate and pregelatinized starch in a white, hard gelatin capsule. In another embodiment the solid dosage form comprises about 75-80% migalastat hydrochloride, about 0.1-2% magnesium stearate and about 20-25% pregelatinized starch. In another specific embodiment, the capsule comprises about 76.5% migalastat hydrochloride, about 0.5% magnesium stearate and about 23% pregelatinized starch.

**Enzyme Replacement Therapy**

The current approved treatment for Fabry disease is enzyme replacement therapy. Two products are currently available for the treatment of Fabry disease: agalsidase alfa (Replagal®, Shire Human Genetic Therapies) and agalsidase beta (Fabrazyme®; Genzyme Corporation), marketed globally. These two forms of ERT are intended to compensate for a patient's inadequate $\alpha$-Gal A activity with a recombinant form of the enzyme, administered intravenously. ERT has been demonstrated to reduce GL-3 deposition in capillary endothelium of the kidney and certain other cell types. While ERT is effective in many settings, the treatment also has limitations. ERT has not been demonstrated to decrease the risk of stroke, cardiac muscle responds slowly, and GL-3 elimination from some of the cell types of the kidneys is limited. Some patients develop immune reactions to ERT.

The recommended dosage of agalsidase alfa is 0.2 mg/kg body weight infused every 2 weeks as an intravenous infusion. A 10-week study was conducted in ERT naive adult males Fabry patients to evaluate the pharmacokinetics and pharmacodynamics of agalsidase alfa. The mean half life after administration of doses ranging from 0.1 to 0.4 mg/kg of agalsidase alfa was 56-76 minutes with no significant association between dose and half life, clearance or volume of distribution. The AUC was linearly proportional to dose over this dose range. Plasma GL-3 levels were reduced in all dose groups by approximately 50%; the reduction was independent of dose and dosing frequency. Two of 18 patients became IgG positive during the study. No IgE antibodies were detected in any patient during the study.

The recommended dosage of agalsidase beta is 1 mg/kg body weight infused every 2 weeks as an intravenous infusion. The manufacturer of agalsidase beta has announced a drug shortage, the only ERT approved in the US for Fabry disease. As a result, agalsidase beta is currently rationed and patients typically receive a reduced dose of the enzyme and/or an extended dosing interval (i.e., greater than 2 weeks between doses). Agalsidase beta exhibits non-linear pharmacokinetics with exposure (AUC) values increasing and clearance decreasing disproportionally with increase in dose. AUC values increased approximately 6-fold and 8-fold when doses were increased from 0.3 mg/kg to 1 mg/kg and from 1 mg/kg to 3 mg/kg, respectively. The elimination half-life of agalsidase beta in adult patients after doses ranging from 0.3 mg/kg to 3 mg/kg was dose dependent and ranged from 45 to 100 minutes.

IgG antibodies to agalsidase beta developed in 79% of adult patients and 69% of pediatric patients treated with agalsidase in clinical studies; the majority of patients who developed IgG antibodies did so within the first 3 months of exposure. Males, particularly those with low residual $\alpha$-Gal A levels, were more likely to develop IgG antibodies than males with higher residual levels or in females. IgG seroconversion in pediatric patients was associated with prolonged half-life of agalsidase. However, in adult patients, identical agalsidase pharmacokinetic profiles were observed before and after seroconversion in one trial; in another trial, maximal agalsidase concentrations and AUC values were reduced up to 26% of baseline values in patients with the highest titers of IgG. The presence of IgG antibodies to agalsidase has been reported to decrease activity of the enzyme.

Migalastat hydrochloride stabilizes wild-type $\alpha$-Gal A in vitro and as well as in vivo. It has been demonstrated in vitro that the binding of migalastat hydrochloride to rha-Gal A resulted in significant time- and concentration-dependent increases in stabilization of rha-Gal A at neutral pH as measured by thermal denaturation and by activity. In a neutral pH buffer, rha-Gal A showed a loss in activity, with a half-life of approximately 3 hours; co-incubation with migalastat hydrochloride increased the half-life for loss of rha-Gal A activity to approximately 40 hours.

In the rat, oral administration of 3 mg/kg of migalastat hydrochloride followed 30 min later by an injection of 10 mg/kg agalsidase beta resulted in a 2.6-fold increase in the plasma half-life of rh$\alpha$-Gal A and a 2.5-fold and 1.5-fold increase in plasma $\alpha$-Gal A levels at 60 and 240 minutes, respectively. In the GLA deficient mouse, oral administration of 30, 100 or 300 mg/kg doses of migalastat hydrochloride 30 min prior to and 2 hours after an injection of rh$\alpha$-Gal A resulted in a dose-dependent increase in tissue $\alpha$-Gal A levels and a dose-dependent reduction in GL-3 levels in skin, heart, kidney, and plasma compared to administration of rh$\alpha$-Gal A alone.

Migalastat hydrochloride has been shown to stabilize agalsidase alfa both in vitro and in vivo. The effect of migalastat hydrochloride on the physical stability of agalsidase alfa was evaluated with an in vitro thermal denaturation assay. Using this assay, agalsidase alfa showed a melting temperature (Tm) of approximately 51°C at pH 7.4. However, when 10 $\mu$M migalastat hydrochloride was included in the denaturation reaction, the Tm of agalsidase alfa was substantially increased to 59°C. As expected for a lysosomal enzyme, agalsidase alfa was more stable at low pH (Tm of 58°C at pH 5.2) and exhibited further resistance to heat-denaturation in the presence of 10 $\mu$M migalastat hydrochloride (Tm of 68°C). These data indicate that binding of migalastat hydrochloride confers a high level of physical stability to agalsidase alfa.

The effect of migalastat hydrochloride on the rate of clearance of agalsidase alfa from the blood of male Sprague-Dawley rats was also investigated. Animals received vehicle (water) or a single oral gavage of 1, 3, 10, or 30 mg/kg migalastat hydrochloride, followed 30 minutes later by intravenous administration of 0.2 mg/kg agalsidase alfa via bolus tail vein injection. Blood was collected as a function of time and $\alpha$-Gal A activity was measured in plasma. In the absence of migalastat hydrochloride, $\alpha$-Gal A activity declined rapidly; pre-administration of migalastat hydrochloride resulted in a dose-dependent increase in the half life of agalsidase alfa (as measured by $\alpha$-Gal A activity) of approximately 2-fold and 3-fold, after administration of 3 mg/kg and 30 mg/kg migalastat hydrochloride, respectively, with an approximately 2.5-fold and 1.5-fold increase in plasma $\alpha$-Gal A levels at 60 and 240 minutes, respectively. The effect of migalastat hydrochloride on agalsidase alfa both in vitro and in vivo is comparable to that observed with migalastat hydrochloride on agalsidase beta.

A preliminary study in GLA deficient mice has been conducted evaluating the safety of co-administered migalastat hydrochloride and Fabrazyme®. Migalastat hydrochloride was administered three times a week for four weeks at doses of 3 and 30 mg/kg in combination with Fabrazyme® administered intravenously once a week at a dose of 1 mg/kg. There appeared to be no direct drug-related changes in survival, clinical condition or hematology and clinical chemistry parameters observed in male GLA-deficient mice co-administered with migalastat hydrochloride and Fabrazyme®.

## EXAMPLES

### EXAMPLE 1: Dosing Regimen for the Treatment of Fabry Disease using Migalastat Hydrochloride and Agalsidase

One objective of the study is to evaluate the safety, effectiveness, and pharmacodynamics of dose regimens comprising co-administering migalastat hydrochloride and agalsidase in patients with Fabry disease.

Another objective of the study is to assess the effects of 150 mg and 450 mg doses of migalastat hydrochloride on the distribution of $\alpha$-Gal A. This will be evaluated by measuring the distribution of agalsidase in skin after dosing with agalsidase alone and agalsidase in combination with migalastat hydrochloride at 24 hours and 7 days after dosing by measuring $\alpha$-Gal A levels and protein levels.

Other measurements that will be evaluated are:

- Urinary GL-3 excretion before and 14 days after each agalsidase dose;
- GL-3 in skin after dosing with agalsidase alone and agalsidase in combination with migalastat hydrochloride at 24 hours and 7 days after dosing;
- WBC $\alpha$-Gal A enzyme levels, determined before initiation of the agalsidase infusion and at 2, 4 and 24 hours and 7 and 14 days after dosing;
- Antibody titer (IgG) before initiation of an infusion of agalsidase;
- Plasma globotriaosylsphingosine (lyso-GB3) concentrations and urinary excretion of lyso-GB3 before each dose of agalsidase and 14 days after each dose of agalsidase.

All plasma, WBC and skin measurements of $\alpha$-Gal A enzyme levels are performed with and without Con A capture and determination of protein levels is by Western blot.

*Study Design.* This is a Phase 2 clinical, two stage, open-label study to assess the safety and effectiveness of co-administering migalastat hydrochloride and agalsidase. The study will be conducted in male subjects between 18 and 65 years of age who have been receiving a stable dose (0.3-1.0 mg/kg) of agalsidase beta (Fabrazyme®) or ($\geq$ 0.2 mg/kg) of agalsidase alfa (Replagal®) at least one month before study entry. Approximately 18 subjects will be enrolled.

This open-label study will consist of two stages. Stage 1 will consist of screening and a three-period study to evaluate the effect of 150 mg migalastat hydrochloride on the pharmacokinetics and safety of agalsidase and the effect of agalsidase on the pharmacokinetics and safety of 150 mg migalastat hydrochloride. Stage 2 will consist of screening and a two-period study to evaluate the effect of 450 mg migalastat hydrochloride on the pharmacokinetics and safety of agalsidase. In Stage 2, the effect of agalsidase on the pharmacokinetics and safety of a 450 mg dose of migalastat hydrochloride will not be evaluated. The plasma exposure of migalastat hydrochloride will be characterized when migalastat hydrochloride is administered with agalsidase solely to confirm the attainment of adequate migalastat hydrochloride plasma concentrations.

Each subject will receive each of the following treatments in the order described below. Stage 1 will consist of the following periods:

Period 1: Agalsidase alone as an intravenous infusion;
Period 2: A 150 mg oral dose of migalastat hydrochloride two hours before initiation of an intravenous infusion of agalsidase;

Period 3: A 150 mg oral dose of migalastat hydrochloride.

The dose of agalsidase administered in Periods 1 and 2 will be identical. Agalsidase alfa will be administered as a 40-minute intravenous infusion and agalsidase beta will be administered as a 2-hour intravenous infusion.

For Period 1, prior to their next scheduled agalsidase infusion, subjects will have the following assessments performed: adverse event assessment, concomitant medications, physical exam, weight, vital signs, 12-lead ECG, clinical laboratory tests (serum chemistry, hematology and urinalysis), skin biopsy (punch biopsy for measurement of α-Gal A enzyme levels; if sufficient sample is available, skin GL-3 will also be determined).

On the morning of Day 1, urine will be collected for urinary GL-3 and lyso-GB3 determinations followed by administration of the subject's current agalsidase dose given as an infusion using an infusion pump. Blood samples for pharmacokinetic and pharmacodynamic analysis will be collected immediately before initiation of the agalsidase infusion and over a 24-hour period after initiation of the agalsidase infusion. Plasma and WBC α-Gal A enzyme levels, plasma lyso-GB3 and plasma antibody titer will be determined from the collected blood samples at the times summarized in Table 2 for agalsidase beta and in Table 4 for agalsidase alfa. A 12-lead ECG will be performed at the end of the agalsidase infusion, immediately after collection of the post-infusion blood sample.

On Day 2, a punch skin biopsy will be collected 24 hours after initiation of the previous day's infusion from which α-Gal A enzyme levels will be determined; if sufficient sample is available, skin GL-3 will also be determined. After collection of the last pharmacokinetic sample, the following assessments will be performed: adverse event assessment, concomitant medications, physical exam, weight, vital signs, and clinical laboratory tests (serum chemistry, hematology, and urinalysis).

On Day 7, subjects will have the following assessments performed: physical exam, vital signs, concomitant medications and adverse event assessment. A skin biopsy will be collected from which α-Gal A enzyme levels will be determined; if sufficient sample is available, skin GL-3 will also be determined. A blood sample for WBC α-Gal A and plasma enzyme level determinations will also be collected. On Day 14, a urine sample for determination of urinary GL-3 and lyso-GB3 excretion will be collected. A blood sample for WBC α-Gal A and plasma enzyme level determinations will also be collected and vital signs assessed.

For Period 2, prior to their next scheduled agalsidase infusion, subjects will have the following assessments performed: adverse event assessment, concomitant medications, physical exam, weight, vital signs, 12-lead ECG, clinical laboratory tests (serum chemistry, hematology and urinalysis).

On the morning of Day 1, urine will be collected for urinary GL-3 and lyso-GB3 determinations followed by administration of an oral dose of 150 mg of migalastat hydrochloride 2 hours prior to the scheduled agalsidase infusion. Subjects will fast for at least 2 hours before and 2 hours after migalastat hydrochloride administration. In Period 2, each subject will receive the identical agalsidase dose administered in Period 1 as an infusion using an infusion pump. The agalsidase infusion will be initiated 2 hours after administration of the migalastat hydrochloride dose.

Blood samples for pharmacokinetic and pharmacodynamic analysis will be collected before dosing migalastat hydrochloride and at 1 hour after administration of migalastat hydrochloride. Additional blood samples will be collected immediately before initiation of the agalsidase infusion and over the 24-hour period after initiation of the agalsidase infusion. Plasma and WBC α-Gal A enzyme levels, plasma lyso-GB3 and plasma antibody titer will be determined from the collected blood samples at the times summarized in Table 2 for agalsidase beta and in Table 4 for agalsidase alfa. A 12-lead ECG will be performed at the end of the agalsidase infusion, immediately after collection of the post-infusion blood sample.

On Day 2, a punch skin biopsy will be collected 24 hours after initiation of the previous day's infusion from which α-Gal A enzyme levels will be determined; if sufficient sample is available, skin GL-3 will also be determined. After collection of the last pharmacokinetic sample, the following assessments will be performed: adverse event assessment, concomitant medications, physical exam, weight, vital signs, and clinical laboratory tests (serum chemistry, hematology, and urinalysis).

On Day 7, subjects will have the following assessments performed: physical exam, vital signs, concomitant medications, and adverse event assessment. A skin biopsy will be collected from which α-Gal A enzyme levels will be determined; if sufficient sample is available, skin GL-3 will also be determined. A blood sample for WBC α-Gal A and plasma enzyme level measurement will also be collected.

On Day 14, a urine sample for determination of urinary GL-3 and lyso-GB3 excretion will be collected. A blood sample for WBC α-Gal A and plasma enzyme level determinations will also be collected and vital signs assessed.

After completing all assessments after Period 2, subjects begin Period 3. All subjects will receive their next agalsidase infusion on Day 1 following their usual dosing schedule. On Day 6, all subjects will have the following assessments performed: adverse event assessment, concomitant medications, physical exam, weight, vital signs, 12-lead ECG, and clinical laboratory tests (serum chemistry, hematology, and urinalysis).

On Day 7, a 150 mg oral dose of migalastat hydrochloride will be administered. Subjects will fast for at least 2 hours before and 2 hours after migalastat hydrochloride administration. Blood samples will be collected before dosing and over the 24-hour period after administration of migalastat hydrochloride. Migalastat hydrochloride concentrations will be measured in all plasma samples (see Table 2 for agalsidase beta and Table 4 for subjects receiving agalsidase alfa for sample collection times).

On Day 8, after collection of the last pharmacokinetic sample, the following assessments will be performed: adverse event assessment, concomitant medications, physical exam, vital signs and clinical laboratory tests (serum chemistry, hematology, and urinalysis).

The follow-up for Period 3 will be by telephone contact 28 days after Period 3. The following assessments will be performed: concomitant medications and adverse events.

For Stage 2, each subject receives each of the following treatments in the order described below:

Period 1: Agalsidase alone as an infusion;
Period 2: A 450 mg oral dose of migalastat hydrochloride two hours before initiation; of an intravenous infusion of agalsidase.

The dose of agalsidase administered in Periods 1 and 2 will be identical. Agalsidase alfa will be administered as a 40-minute intravenous infusion and agalsidase beta will be administered as a 2-hour intravenous infusion.

For Period 1 subjects meeting all eligibility criteria will, prior to their next scheduled agalsidase infusion, have the following assessments performed: adverse event assessment, concomitant medications, physical exam, weight, vital signs, 12-lead ECG, clinical laboratory tests (serum chemistry, hematology and urinalysis), skin biopsy (punch biopsy for measurement of $\alpha$-Gal A enzyme levels; if sufficient sample is available, skin GL-3 will also be determined).

On the morning of Day 1, urine will be collected for urinary GL-3 and lyso-GB3 determinations followed by administration of the subject's current agalsidase dose given as an infusion using an infusion pump. Blood samples for pharmacokinetic and pharmacodynamic analysis will be collected immediately before initiation of agalsidase infusion and over the 24-hour period after initiation of the agalsidase infusion. Plasma and WBC $\alpha$-Gal A enzyme levels, plasma lyso-GB3 and plasma antibody titer will be determined from the collected blood samples at the times summarized in Table 3 for agalsidase beta and in Table 5 for agalsidase alfa. A 12-lead ECG will be performed at the end of the agalsidase infusion, immediately after collection of the post-infusion blood sample.

On Day 2, a punch skin biopsy will be collected 24 hours after initiation of the previous day's infusion from which $\alpha$-Gal A enzyme levels will be determined; if sufficient sample is available, skin GL-3 will also be determined. After collection of the last pharmacokinetic sample, the following assessments will be performed: adverse event assessment, concomitant medications, physical exam, weight, vital signs, and clinical laboratory tests (serum chemistry, hematology, and urinalysis).

On Day 7, subjects will have the following assessments performed: vital signs, concomitant medications and adverse event assessment. A skin biopsy will be collected from which $\alpha$-Gal A enzyme levels will be determined; if sufficient sample is available, skin GL-3 will also be determined. A blood sample for WBC $\alpha$-Gal A and plasma enzyme level measurement will also be collected.

On Day 14, a urine sample for determination of urinary GL-3 and lyso-GB3 excretion will be collected. A blood sample for WBC $\alpha$-Gal A and plasma enzyme level determinations will also be collected and vital signs assessed.

For Period 2, subjects, prior to their next scheduled agalsidase infusion, will have the following assessments performed: adverse event assessment, concomitant medications, physical exam, weight, vital signs, 12-lead ECG, clinical laboratory tests (serum chemistry, hematology and urinalysis).

On the morning of Day 1, urine will be collected for urinary GL-3 and lyso-GB3 determinations followed by administration of an oral dose of 450 mg of migalastat hydrochloride 2 hours prior to the scheduled agalsidase infusion. Subjects will fast for at least 2 hours before and 2 hours after migalastat hydrochloride administration. In Period 2, each subject will receive the identical agalsidase dose administered in Period 1 as an infusion using an infusion pump. The agalsidase infusion will be initiated 2 hours after administration of the migalastat hydrochloride dose.

Blood samples for pharmacokinetic and pharmacodynamic analysis will be collected pre-migalastat hydrochloride dose and at 1 hour after administration of migalastat hydrochloride. Additional blood samples will be collected immediately before initiation of the agalsidase infusion and over the 24-hour period after initiation of the agalsidase infusion. Plasma and WBC $\alpha$-Gal A enzyme levels, plasma lyso-GB3 and plasma antibody titer will be determined from the collected blood samples at the times summarized in Table 3 for agalsidase beta and in Table 5 for agalsidase alfa. A 12-lead ECG will be performed at the end of the agalsidase infusion, immediately after collection of the post-infusion blood sample.

On Day 2, a punch skin biopsy will be collected 24 hours after initiation of the previous day's infusion from which $\alpha$-Gal A enzyme levels will be determined; if sufficient sample is available, skin GL-3 will also be determined. After collection of the last pharmacokinetic sample, the following assessments will be performed: adverse event assessment, concomitant medications, physical exam, weight, vital signs, and clinical laboratory tests (serum chemistry, hematology, and urinalysis).

On Day 7, subjects will have the following assessments performed: vital signs, concomitant medications, and adverse event assessment. A skin biopsy will be collected from which α-Gal A enzyme levels will be determined. A blood sample for WBC α-Gal A and plasma enzyme level measurement will also be collected.

On Day 14, a urine collection for determination of urinary GL-3 and lyso-GB3 excretion will be performed. A blood sample for WBC α-Gal A and plasma enzyme level determinations will also be collected and vital signs assessed.

The follow-up will be 28 days after Period 2. The following assessments will be performed: concomitant medications and adverse events.

*Assessment and Sample Collection Schedules.* Table 1 shows the schedule of assessments for Stages 1 and 2. Sample collection times and analytes for co-administration of migalastat hydrochloride with Fabrazyme® are shown in Tables 2 and 3. Sample collection times and analytes for co-administration of migalastat hydrochloride with Replagal® are shown in Tables 4 and 5.

**Table 1: Schedule of Assessments (Stages 1 and 2)**

| Activity | Screening | Period 1 and 2 (Stages 1 and 2) | | | | | Period 3 (Stage 1 only) | | | | Follow-up (Stages 1 and 2) |
|---|---|---|---|---|---|---|---|---|---|---|---|
| | Within 28 days of Enrollment | Day -1 | Day 1 | Day 2 | Day 7 | Day 14 | Day 1 | Day 6 | Day 7 | Day 8 | Telephone Contact |
| Informed Consent | X | | | | | | | | | | |
| Medical History and Demographic Data | X | | | | | | | | | | |
| Physical Exam | X | X | | X | X | | | X | | X | |
| ECG (12-lead) | X | X | X | | | | | X | X | | |
| Vital Signs[6] | X | X | X | X | X | X | | X | X | X | |
| Hematology | X | X | | X | | | | x | | X | |
| Urinalysis | X | X | | X | | | | X | | X | |
| Serum Chemistry | X | X | | X | | | | X | | X | |
| Record of Concomitant Medication | X | X | X | X | X | X | | X | X | X | X |
| eGFR | X | | | | | | | | | | |
| Urine GL-3/LysoGB3 | | X | | | | X | | | | | |
| Check-in Procedures | | X | | | | | | X | | | |
| Drug Dose | | | X[1] | | | | X[2] | | X[3] | | |
| PK Blood Sampling[4] | | | X | X | X | X | | | X | X | |
| Skin Biopsy[4] | | X[5] | | X | X | | | | | | |
| WBC Collection[4] | | | X | X | X | X | | | | | |
| Antibody Titer[4] | | X | | | | | | | | | |

(continued)

| Activity | Screening | Period 1 and 2 (Stages 1 and 2) | | | | | Period 3 (Stage 1 only) | | | | Follow-up (Stages 1 and 2) |
|---|---|---|---|---|---|---|---|---|---|---|---|
| | Within 28 days of Enrollment | Day -1 | Day 1 | Day 2 | Day 7 | Day 14 | Day 1 | Day 6 | Day 7 | Day 8 | Telephone Contact |
| Adverse Events | | X | X | X | X | X | | X | X | X | X |

1Drug(s) administered: agalsidase (Periods 1 and 2), migalastat HCl (Period 2) 2 Outpatient administration of Fabrazyme® or Replagal® 3 Drug administered: migalastat HCl 4 Sample collection times and analytes summarized in Table 2 and Table 3 for **Fabrazyme**® (agalsidase beta) and in Table 4 and Table 5 for **Replagal**® (agalsidase alfa).

5 Period 1 only.

6 Vital signs include temperature, blood pressure, heart rate and respiration.

**Table 2:** FABRAZYME (agalsidase beta) - Stage 1:  Blood, Urine and Skin Collection Times and Sample Analysis

**Period 1**  Collection Schedule

| Sample Collected | Post-Initiation of Fabrazyme® Infusion (hr) | | | | | | | | | | | | | | Day | |
|---|---|---|---|---|---|---|---|---|---|---|---|---|---|---|---|---|
| | 0 | 0.5 | 1 | 1.5 | 2 | 2.5 | 3 | 4 | 5 | 6 | 7 | 8 | 12 | 24 | 7 | 14 |
| Blood | X | X | X | X | X | X | X | X | X | X | X | X | X | X | X | X |
| Skin | X | | | | | | | | | | | | | X | X | |
| Urine | X | | | | | | | | | | | | | | | X |

Fabrazyme® Infusion ⟵⟶

**Sample Analysis**

| | | Post-Initiation of Fabrazyme® Infusion (hr) | | | | | | | | | | | | | | Day | |
|---|---|---|---|---|---|---|---|---|---|---|---|---|---|---|---|---|---|
| | | 0 | 0.5 | 1 | 1.5 | 2 | 2.5 | 3 | 4 | 5 | 6 | 7 | 8 | 12 | 24 | 7 | 14 |
| Plasma | Plasma α-Gal A | X | X | X | X | X | X | X | X | X | X | X | X | X | X | X | X |
| | Antibody | X | | | | | | | | | | | | | | | |
| | WBC α-Gal A | X | | | | X | | | X | | | | | | X | X | X |
| | Lyso-GB3 | X | | | | | | | | | | | | | | X | X |
| Skin | α-Gal A * | X | | | | | | | | | | | | | X | X | |
| Urine | GL-3 | X | | | | | | | | | | | | | | | X |
| | Lyso-GB3 | X | | | | | | | | | | | | | | | X |

**Period 2**  Collection Schedule

| Sample Collected | Post-Migalastat HCl | | Post-Initiation of Fabrazyme® Infusion (hr) | | | | | | | | | | | | | | | Day | |
|---|---|---|---|---|---|---|---|---|---|---|---|---|---|---|---|---|---|---|---|
| | 0 | 1 | 0 | 0.5 | 1 | 1.5 | 2 | 2.5 | 3 | 4 | 5 | 6 | 7 | 8 | 10 | 12 | 24 | 7 | 14 |
| Blood | X | X | X | X | X | X | X | X | X | X | X | X | X | X | X | X | X | X | X |
| Skin | | | | | | | | | | | | | | | | | X | X | |
| Urine | X | | | | | | | | | | | | | | | | | | X |
| Migalastat HCl Administration | X | | | | | | | | | | | | | | | | | | |

Fabrazyme®Infusion (arrow)  ⟵⟶

**Sample Analysis**

| Plasma | Plasma α-Gal A | X | | X | X | X | X | X | X | X | X | X | X | X | X | | X | X | | X | X |
|---|---|---|---|---|---|---|---|---|---|---|---|---|---|---|---|---|---|---|---|---|---|
| | Migalastat HCl | X | X | X | | X | | X | | X | X | X | X | | X | X | | X | | X | |
| | Antibody | X | | | | | | | | | | | | | | | | | | | |
| | WBC α-Gal A | X | | | | | X | | X | | | | | | | | X | | X | X | X |
| | Lyso-GB3 | X | | | | | | | | | | | | | | | | | | X | X |
| Skin | α-Gal A * | | | | | | | | | | | | | | | | X | | X | | |
| Urine | GL-3 | X | | | | | | | | | | | | | | | | | | | X |
| | Lyso-GB3 | X | | | | | | | | | | | | | | | | | | | X |

**Period 3**                                    **Collection Schedule**

**Post Migalastat HCl Dose**

| Sample Collected | 0 | 1 | 2 | 3 | 4 | 5 | 6 | 7 | 8 | 10 | 12 | 24 |
|---|---|---|---|---|---|---|---|---|---|---|---|---|
| Blood | X | X | X | X | X | X | X | X | X | X | X | X |
| Skin | | | | | | | | | | | | |
| Urine | | | | | | | | | | | | |
| Migalastat HCl Administration | X | | | | | | | | | | | |

**Sample Analysis**

| Plasma | Migalastat HCl | X | X | X | X | X | X | X | X | X | X | X | X |
|---|---|---|---|---|---|---|---|---|---|---|---|---|---|

\* If sufficient sample is available, skin GL-3 will also be measured.

**Table 3:    FABRAZYME (agalsidase beta) - Stage 2 - Blood, Urine and Skin Collection Times and Sample Analysis**

**Period 1**                                    **Collection Schedule**

| | Post-Initiation of Fabrazyme® Infusion (hr) | | | | | | | | | | | | | | Day | |
|---|---|---|---|---|---|---|---|---|---|---|---|---|---|---|---|---|
| Sample Collected | 0 | 0.5 | 1 | 1.5 | 2 | 2.5 | 3 | 4 | 5 | 6 | 7 | 8 | 12 | 24 | 7 | 14 |
| Blood | X | X | X | X | X | X | X | X | X | X | X | X | X | X | X | X |
| Skin | X | | | | | | | | | | | | | X | X | |
| Urine | X | | | | | | | | | | | | | | | X |
| Fabrazyme® Infusion | ← | | → | | | | | | | | | | | | | |

**Sample Analysis**

| Plasma | Plasma α-Gal A | X | X | X | X | X | X | X | X | X | X | X | X | X | X | X | X |
|---|---|---|---|---|---|---|---|---|---|---|---|---|---|---|---|---|---|
| | Antibody | X | | | | | | | | | | | | | | | |
| | WBC α-Gal A | X | | | | X | | | X | | | | | | X | X | X |
| | Lyso-GB3 | X | | | | | | | | | | | | | | X | X |
| Skin | α-Gal A * | X | | | | | | | | | | | | | X | X | |
| Urine | GL-3 | X | | | | | | | | | | | | | | | X |
| | Lyso-GB3 | X | | | | | | | | | | | | | | | X |

14

**Period 2**                                    **Collection Schedule**

| Sample Collected | Post-Migalastat HCl 0 | Post-Migalastat HCl 1 | Fabrazyme 0 | 0.5 | 1 | 1.5 | 2 | 2.5 | 3 | 4 | 5 | 6 | 7 | 8 | 10 | 12 | 24 | Day 7 | Day 14 |
|---|---|---|---|---|---|---|---|---|---|---|---|---|---|---|---|---|---|---|---|
| Blood | X | X | X | X | X | X | X | X | X | X | X | X | X | X | X | X | X | X | X |
| Skin | | | | | | | | | | | | | | | | | X | X | |
| Urine | X | | | | | | | | | | | | | | | | | | X |
| Migalastat HCl Administration Fabrazyme® Infusion (arrow) | X | | | ←———————→ | | | | | | | | | | | | | | | |

**Sample Analysis**

| | | 0 | 1 | 0 | 0.5 | 1 | 1.5 | 2 | 2.5 | 3 | 4 | 5 | 6 | 7 | 8 | 10 | 12 | 24 | Day 7 | Day 14 |
|---|---|---|---|---|---|---|---|---|---|---|---|---|---|---|---|---|---|---|---|---|
| Plasma | Plasma α-Gal A | X | | X | X | X | X | X | X | X | X | X | X | X | X | | X | X | X | X |
| | Migalastat HCl | X | X | X | | X | | X | | X | X | X | X | | X | X | | X | | |
| | Antibody | X | | | | | | | | | | | | | | | | | | |
| | WBC α-Gal A | X | | | | | | X | | | X | | | | | | | X | X | X |
| | Lyso-GB3 | X | | | | | | | | | | | | | | | | | X | X |
| Skin | α-Gal A * | | | | | | | | | | | | | | | | | X | X | |
| Urine | GL-3 | X | | | | | | | | | | | | | | | | | | X |
| | Lyso-GB3 | X | | | | | | | | | | | | | | | | | | X |

\* If sufficient sample is available, skin GL-3 will also be measured.

**Table 4:**     **REPLAGAL (agalsidase alfa) - Stage 1: Blood, Urine and Skin Collection Times and Sample Analysis**

**Period 1**                                   **Collection Schedule**

| Sample Collected | Post-Initiation of Replagal® Infusion (hr) 0 | 0.33 | 0.66 | 1 | 1.5 | 2 | 3 | 4 | 5 | 6 | 7 | 8 | 12 | 24 | Day 7 | Day 14 |
|---|---|---|---|---|---|---|---|---|---|---|---|---|---|---|---|---|
| Blood | X | X | X | X | X | X | X | X | X | X | X | X | X | X | X | X |
| Skin | X | | | | | | | | | | | | | X | X | |
| Urine | X | | | | | | | | | | | | | | | X |
| Replagal® Infusion | ←———————→ | | | | | | | | | | | | | | | |

**Sample Analysis**

| | | 0 | 0.33 | 0.66 | 1 | 1.5 | 2 | 3 | 4 | 5 | 6 | 7 | 8 | 12 | 24 | Day 7 | Day 14 |
|---|---|---|---|---|---|---|---|---|---|---|---|---|---|---|---|---|---|
| Plasma | Plasma α-Gal A | X | X | X | X | X | X | X | X | X | X | X | X | X | X | X | X |
| | Antibody | X | | | | | | | | | | | | | | | |
| | WBC α-Gal A | X | | | | | X | | X | | | | | | X | X | X |
| | Lyso-GB3 | X | | | | | | | | | | | | | | X | X |
| Skin | α-Gal A * | X | | | | | | | | | | | | | X | X | |
| Urine | GL-3 | X | | | | | | | | | | | | | | | X |
| | Lyso-GB3 | X | | | | | | | | | | | | | | | X |

**Period 2**                                                        **Collection Schedule**

| Sample Collected | Post-Migalastat HCl 0 | 1 | Post-Initiation of Replagal® Infusion (hr) 0 | 0.33 | 0.66 | 1 | 1.5 | 2 | 3 | 4 | 5 | 6 | 7 | 8 | 10 | 12 | 24 | Day 7 | 14 |
|---|---|---|---|---|---|---|---|---|---|---|---|---|---|---|---|---|---|---|---|
| Blood | X | X | X | X | X | X | X | X | X | X | X | X | X | X | X | X | X | X | X |
| Skin |  |  |  |  |  |  |  |  |  |  |  |  |  |  |  |  | X | X |  |
| Urine | X |  |  |  |  |  |  |  |  |  |  |  |  |  |  |  |  |  | X |
| Migalastat HCl Administration | X | | ← | | | → | | | | | | | | | | | | | |
| Replagal® Infusion (arrow) | | | | | | | | | | | | | | | | | | | |

**Sample Analysis**

| Plasma | Plasma α-Gal A | X |  | X | X | X | X | X | X | X | X | X | X | X |  | X | X | X | X |
|---|---|---|---|---|---|---|---|---|---|---|---|---|---|---|---|---|---|---|---|
|  | Migalastat HCl | X | X | X |  |  | X |  | X | X | X | X | X |  | X | X |  | X |  |  |
|  | Antibody | X |  |  |  |  |  |  |  |  |  |  |  |  |  |  |  |  |  |  |
|  | WBC α-Gal A | X |  |  |  |  |  | X |  | X |  |  |  |  |  |  | X | X | X |  |
|  | Lyso-GB3 | X |  |  |  |  |  |  |  |  |  |  |  |  |  |  |  | X | X |  |
| Skin | α-Gal A * |  |  |  |  |  |  |  |  |  |  |  |  |  |  |  | X | X |  |  |
| Urine | GL-3 | X |  |  |  |  |  |  |  |  |  |  |  |  |  |  |  |  |  | X |
|  | Lyso-GB3 | X |  |  |  |  |  |  |  |  |  |  |  |  |  |  |  |  |  | X |

**Period 3**                              **Collection Schedule**

**Post Migalastat HCl Dose**

| Sample Collected | 0 | 1 | 2 | 3 | 4 | 5 | 6 | 7 | 8 | 10 | 12 | 24 |
|---|---|---|---|---|---|---|---|---|---|---|---|---|
| Blood | X | X | X | X | X | X | X | X | X | X | X | X |
| Skin |  |  |  |  |  |  |  |  |  |  |  |  |
| Urine |  |  |  |  |  |  |  |  |  |  |  |  |
| Migalastat HCl Administration | X |  |  |  |  |  |  |  |  |  |  |  |

**Sample Analysis**

| Plasma | Migalastat HCl | X | X | X | X | X | X | X | X | X | X | X | X |
|---|---|---|---|---|---|---|---|---|---|---|---|---|---|

\* If sufficient sample is available, skin GL-3 will also be measured.

**Table 5:    REPLAGAL (agalsidase alfa) – Stage 2: Blood, Urine and Skin Collection Times and Sample Analysis**

**Period 1**                              **Collection Schedule**

| Sample Collected | Post-Initiation of Replagal® Infusion (hr) 0 | 0.33 | 0.66 | 1 | 1.5 | 2 | 3 | 4 | 5 | 6 | 7 | 8 | 12 | 24 | Day 7 | 14 |
|---|---|---|---|---|---|---|---|---|---|---|---|---|---|---|---|---|
| Blood | X | X | X | X | X | X | X | X | X | X | X | X | X | X | X | X |
| Skin | X |  |  |  |  |  |  |  |  |  |  |  |  | X | X |  |
| Urine | X |  |  |  |  |  |  |  |  |  |  |  |  |  |  |  | X |
| Replagal® Infusion | | ← | | | → | | | | | | | | | | | |

**Sample Analysis**

| | | | | | | | | | | | | | | | | | | |
|---|---|---|---|---|---|---|---|---|---|---|---|---|---|---|---|---|---|---|
| Plasma | Plasma α-Gal A | X | X | X | X | X | X | X | X | X | X | X | X | X | X | | X | X |
| | Antibody | X | | | | | | | | | | | | | | | | |
| | WBC α-Gal A | X | | | | X | | X | ╱ | | | | | | X | | X | X |
| | Lyso-GB3 | X | | | | | | | | | | | | | | | X | X |
| Skin | α-Gal A * | X | | | | | | | | | | | | X | | | X | |
| Urine | GL-3 | X | | | | | | | | | | | | | | | | X |
| | Lyso-GB3 | X | | | | | | | | | | | | | | | | X |

**Period 2**    **Collection Schedule**

| Sample Collected | Post-Migalastat HCl | | Post-Initiation of Replagal® Infusion (hr) | | | | | | | | | | | | | | | Day | |
|---|---|---|---|---|---|---|---|---|---|---|---|---|---|---|---|---|---|---|---|
| | 0 | 1 | 0 | 0.33 | 0.66 | 1 | 1.5 | 2 | 3 | 4 | 5 | 6 | 7 | 8 | 10 | 12 | 24 | 7 | 14 |
| Blood | X | X | X | X | X | X | X | X | X | X | X | X | X | X | X | X | X | X | X |
| Skin | | | | | | | | | | | | | | | | | X | X | |
| Urine | X | | | | | | | | | | | | | | | | | | X |
| Migalastat HCl Administration | X | | ←————————————→ | | | | | | | | | | | | | | | | |

Fabrazyme® Infusion (arrow)

**Sample Analysis**

| | | | | | | | | | | | | | | | | | | | | |
|---|---|---|---|---|---|---|---|---|---|---|---|---|---|---|---|---|---|---|---|---|
| Plasma | Plasma α-Gal A | X | | X | X | X | X | X | X | X | X | X | X | X | | X | X | | X | X |
| | Migalastat HCl | X | X | X | | | X | | X | X | X | X | X | | X | X | | X | | |
| | Antibody | X | | | | | | | | | | | | | | | | | | |
| | WBC α-Gal A | X | | | | X | | X | | | | | | | | | | X | X | X |
| | Lyso-GB3 | X | | | | | | | | | | | | | | | | | X | X |
| Skin | α-Gal A * | | | | | | | | | | | | | | | | X | | X | |
| Urine | GL-3 | X | | | | | | | | | | | | | | | | | | X |
| | Lyso-GB3 | X | | | | | | | | | | | | | | | | | | X |

**\* If sufficient sample is available, skin GL-3 will also be measured.**

*Migalastat HCl and rha-Gal A Pharmacokinetics.* Concentrations of migalastat HCl in blood samples will be measured in plasma using a validated LC-MS/MS assay. α-Gal A levels in plasma will be determined by a validated assay measuring enzyme activity using 4-MUG, with and without Con A. α-Gal A protein levels will be measured by Western blotting using anti-human Gal A antibody.

*α-Gal A Enzyme Levels in Skin.* α-Gal A enzyme levels will be examined in skin biopsy samples. Skin biopsies will be done using a "punch" device. One piece will be removed at each visit. α-Gal A levels in skin will be determined by a validated assay measuring enzyme activity using 4-MUG, with and without Con A. α-Gal A protein levels will be measured by Western blotting using anti-human Gal A antibody.

*WBC α-Gal A Levels.* α-Gal A levels in WBCs will be determined in blood samples by a validated assay measuring enzyme activity using 4-MUG, with and without Con A. α-Gal A protein levels will be measured by Western blotting using anti-human Gal A antibody.

*Plasma Lyso-GB3.* Measurements of plasma lyso-GB3 will be performed on an exploratory basis to obtain data in patients receiving ERT alone and ERT with co-administered migalastat hydrochloride. Concentrations of lyso-GB3 will be measured in plasma using a validated assay.

*Urine GL-3 and Lyso-GB3.* A first in morning void urine sample will be collected from each subject for analysis of urine GL-3 and lyso-GB3 excretion on Day 1 and Day 14 of Periods 1 and 2. The subjects collect urine in the morning of Days 1 and 14. Urinary GL-3 and urinary Lyso-GB3 will be expressed as a function of urinary creatinine concentration.

*Antibody Titer.* Blood samples will be collected and IgG antibody titers will be measured in each blood sample.

*Safety Parameters.* Safety parameters will be assessed by review of changes in physical exam findings, vital signs, ECG changes over time, clinical labs and adverse events.

*Vital Signs, Weight and Height.* Body temperature and respirations will be measured at screening and check-in. To monitor safety, body temperature, respiration, seated blood pressure and heart rate will be measured before dosing and approximately 1, 2, 3, 4, and 6 hours following administration of agalsidase (Period 1) or migalastat hydrochloride (Periods 2 and 3), on Days 2, 7 and 14. Where the time of vital sign monitoring coincides with a blood draw, the blood draw takes precedence and the vital signs will be adjusted accordingly.

*ECG Monitoring.* ECG monitoring will be performed with a standard 12-lead ECG.

*Clinical Laboratory Tests.* Blood samples for clinical laboratory tests (hematology, serum chemistry) and urinalyses will be collected at every visit and analyzed at the central laboratory. Hematology tests include total hemoglobin, hematocrit, erythrocyte, platelet and leukocyte counts with differential.

- Coagulation (screening only) includes INR and aPTT.
- Serum chemistry includes measurement of AST, ALT, alkaline phosphatase, total bilirubin, creatinine, urea, glucose, calcium, sodium, potassium, magnesium, total protein, albumin, bicarbonate, LDH, blood urea nitrogen, chloride, and phosphate. Measurement of serum creatinine will be performed using reagents that have been calibrated to an isotope dilution mass spectrometry (IDMS) reference method.
- Urinalysis includes color, appearance, specific gravity, pH, protein, glucose, ketones, blood, leukocyte esterase, nitrite, bilirubin, urobilinogen and microscopy of sediment.

*Pharmacokinetic Parameters.* Non-compartmental pharmacokinetic parameters of $AUC_{0-t}$, $AUC_{infinity}$, $C_{max}$, $t_{max}$, $k_{el}$ and half-life will be calculated from plasma migalastat hydrochloride concentrations and $\alpha$-Gal A enzyme levels. *Pharmacokinetic* parameters will be summarized by treatment using descriptive statistics. The $AUC_{0-t}$, $AUC_{infinity}$ ratios for each compound alone to the respective compound in combination will be calculated. Pharmacokinetic and pharmacodynamic data for those subjects receiving agalsidase alfa and agalsidase beta will be analyzed separately.

*Statistical Analysis.* The descriptive statistics (N, mean, standard deviation, and coefficient of variation, standard error, median, minimum and maximum) will be provided as appropriate. The effect of a compound on the co-administered compound will be evaluated by calculation of the individual (by subject) AUC and $C_{max}$ ratios as follows:

$$\text{AUC Ratio} = \frac{AUC_{infinity \, (combination)}}{AUC_{infinity(alone)}}$$

$$\text{Cmax Ratio} = \frac{C_{max \, (combination)}}{C_{max(alone)}}$$

The AUC and $C_{max}$ ratios will be expressed as a mean of the individual ratios and 90% confidence interval for the mean. Pharmacokinetic and pharmacodynamic data for those subjects receiving agalsidase alfa and agalsidase beta will be analyzed separately. Results will be presented in tabular and graphic forms, as appropriate. All subjects who will be dosed with study medication and have sufficient data to generate reliable pharmacokinetic parameters will be included in the safety and pharmacokinetic analysis.

**EXAMPLE 2: Dosing Regimen for the Treatment of Fabry Disease using Migalastat Hydrochloride and Agalsidase**

Migalastat HCl is a pharmacological chaperone for $\alpha$-galactosidase A (a-Gal A) which increases the enzyme's stability and proper folding. Migalastat may act by preventing $\alpha$-Gal A inactivation by stabilizing the enzyme in the pH/temperature conditions of the blood. The objective for this study was to characterize the effects of 150 mg and 450 mg migalastat administered 2 hours before administration of agalsidase on the safety and plasma pharmacokinetics of agalsidase in subjects with Fabry Disease. The objective for this study was also to characterize the effects of 150 mg and 450 mg migalastat on the plasma, skin, and PBMC pharmacokinetics of agalsidase in patients with Fabry Disease; to characterize the effect of plasma agalsidase on the plasma pharmacokinetics of migalastat; to evaluate urine GL-3 and plasma and urine lyso-GB3 prior to and 14 days post agalsidase infusion; and to assess antibody titer prior to agalsidase infusion.

*Methods.* The study was conducted according to the methods described in Example 1. Specifically, this was an open-label, single dose, non-randomized, fixed-sequence, 2-stage study. Stage 1 comprised of 3 periods:

(1) agalsidase beta 0.5 mg/kg or 1.0 mg/kg (infusion for about 2 hours) or agalsidase alpha 0.2 mg/kg (infusion for about 40 min.) enzyme replacement montherapy (ERT);
(2) ERT + 150 mg migalastat oral tablet (single dose) co-administration, migalastat administered 2 hrs. prior to ERT; and
(3) 150 mg migalastat monotherapy.

Stage 2 was comprised of 2 periods in the same sequence as Stage 1 (*i.e.*, Periods (1) and (2)), but with 3x150 mg migalastat oral tablets co-administered in Period 2 (*i.e.*, 450 mg migalastat). Stage 2 comprised of the following 2 periods:

(1) agalsidase beta 0.5 mg/kg or 1.0 mg/kg (infusion for about 2 hours) or agalsidase alpha 0.2 mg/kg (infusion for about 40 min.) enzyme replacement montherapy (ERT); and
(2) ERT + 450 mg migalastat oral tablets co-administration. Migalastat administered 2 hrs. prior to ERT.

Treatment periods were separated by a minimum 14-day washout period.

α-Gal A catalyzes the initial step in breakdown of substrate GL-3 in vivo. α-Gal A also acts on other substrates with the same α-bond such as the artificial low-molecular weight florescent substrate, 4-MUG. α-Gal A activity on 4-MUG was measured in vitro from plasma, skin, and PBMC samples following serial dilutions to dissociate migalastat.

6 patients with plasma, skin, and PBMC α-galactosidase A activity from Stage 1, Periods 1 and 2, were evaluated. Four patients received 0.5 mg/kg agalsidase beta ERT and 2 patients received 1.0 mg/kg agalsidase beta ERT during Period 1, and co-administration of 150 mg migalastat 2 hours prior to initiation of the same dose of ERT during Period 2. Duration of infusion was 2 hours for both periods with one exception: one patient receiving 0.5 mg/kg agalsidase beta had an unbalanced infusion, wherein the patient was infused for 2 hrs and 40 min during Period 2, but was infused for only 2 hrs during Period 1.

***Results:***

Plasma α-Gal-A Activity Increases with Co-Administration of migalastat

For co-administration with migalastat (Period 2) relative to ERT alone (Period 1), the following mean increases in plasma α-Gal A activity AUC (Area Under the Curve) were observed:

Mean increases for the 0.5 mg/kg agalsidase beta infusion:

- 3.0-fold for 0.5 mg/kg agalsidase beta (N=4)

  - Individual patient increases: 2.0-, 2.2-, 3.4-, and 4.2-fold
  - The mean increase excluding the 2.0-fold patient with the unbalanced infusion duration: 3.3-fold

Mean increases for the 1.0 mg/kg agalsidase beta infusion:

- 1.9-fold for 1.0 mg/kg agalsidase beta (N=2)
  - Individual patient increases: 1.6- and 2.2-fold

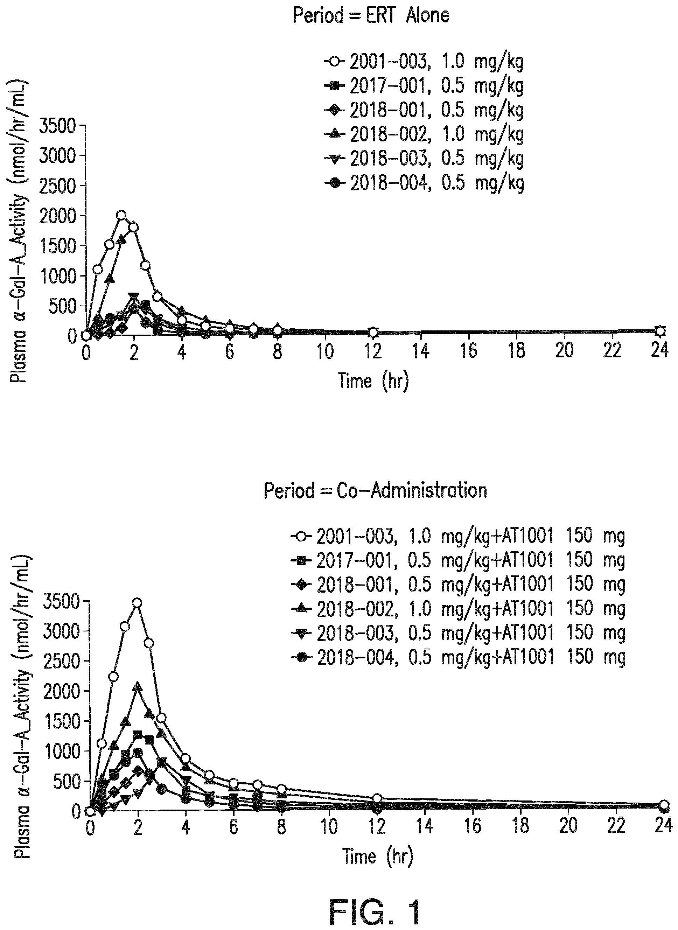

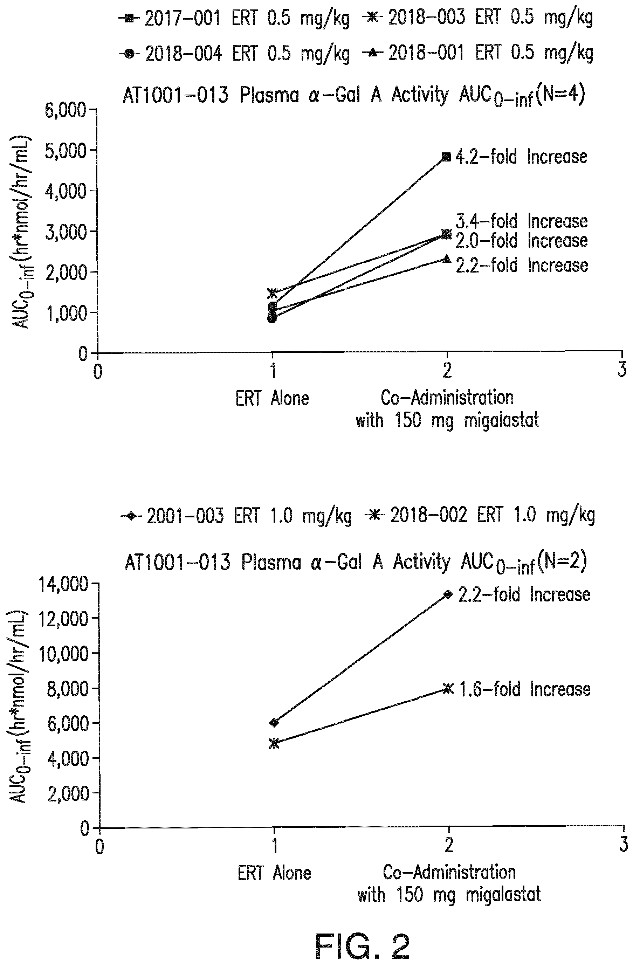

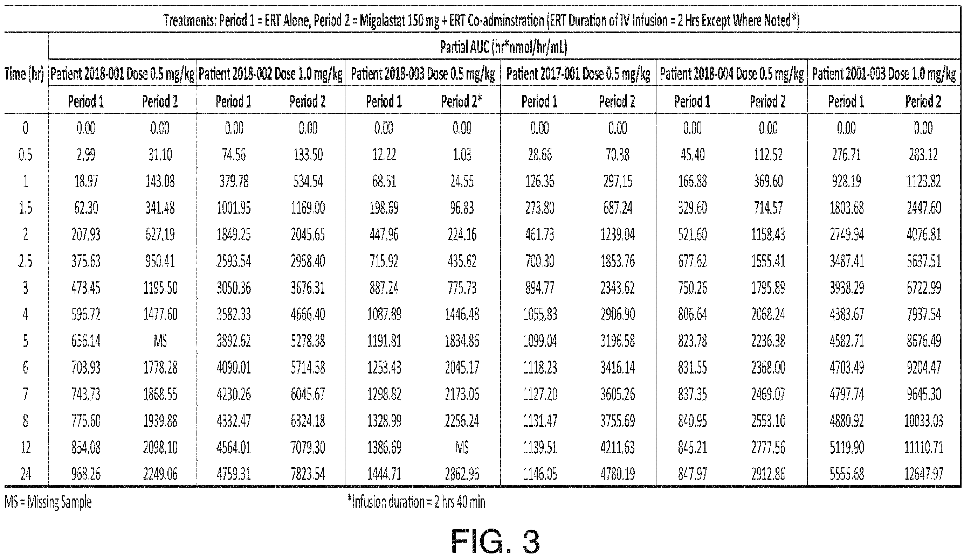

Plasma α-Gal A activity composites of the six patients for Periods 1 and 2 are shown in Figure 1. Plasma α-Gal A activity AUC increases for all patients following co-administration with migalastat is shown in Figure 2. Figure 3 shows the partial AUC's for each sampling time which show increased activity plasma α-Gal A activity with co-administration of 0.5 mg/kg or 1.0 mg/kg agalsidase beta and 150 mg migalastat.

Skin α-Gal-A Activity Increases with Migalastat

For co-administration with migalastat (Period 2) relative to ERT alone (Period 1), the following mean increases in skin α-Gal A activity were observed:

Mean increases for the 0.5 mg/kg agalsidase beta infusion:

- 2.6-fold for 0.5 mg/kg agalsidase beta on Day 2 (N=3, 1 patient had a lost sample)

  - Individual patient increases: 2.8-, 3.9-, and 1.1-fold
  - No change in skin activity from Period 1 on Day 7

Mean increases for the 1.0 mg/kg agalsidase beta infusion:

- 1.9- and 1.5-fold for 1.0 mg/kg agalsidase beta on Days 2 and 7, respectively (N=2)

▪ Individual patient increases: 1.6- and 2.1-fold on Day 2, 1.7- and 1.2-fold on Day 7

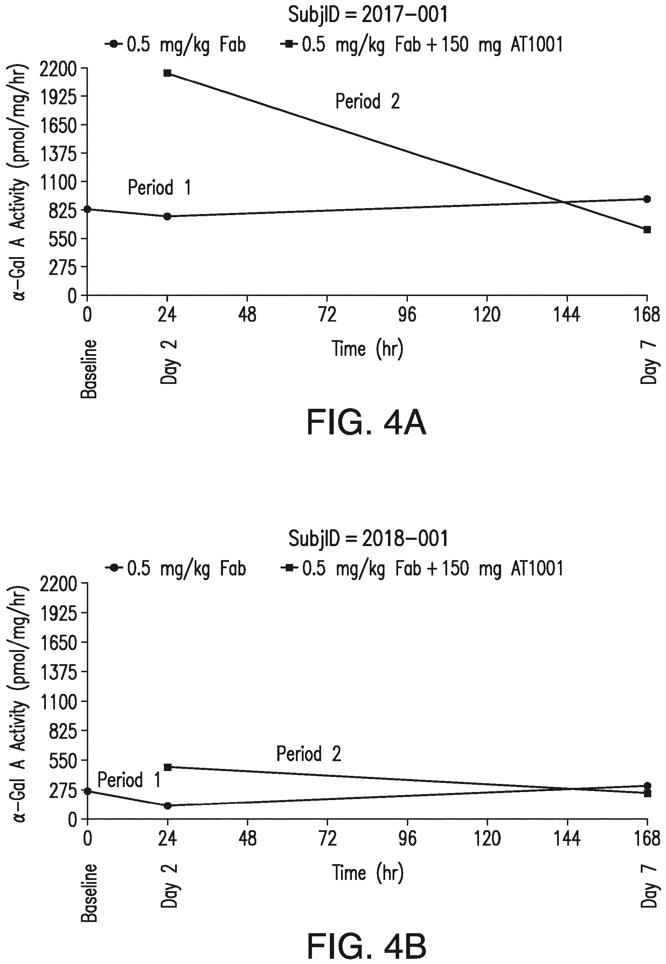

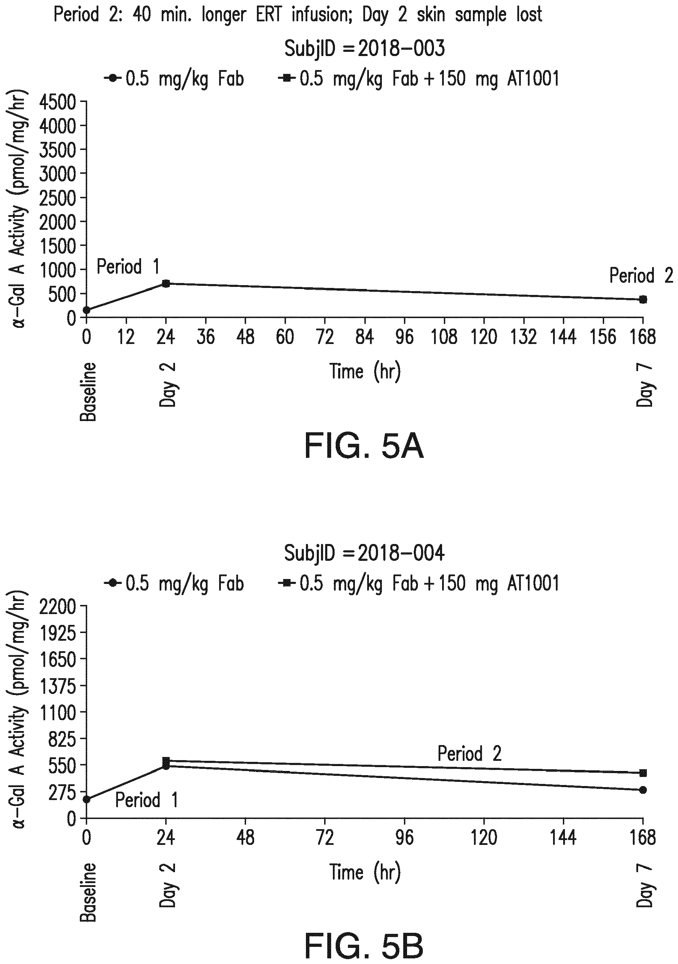

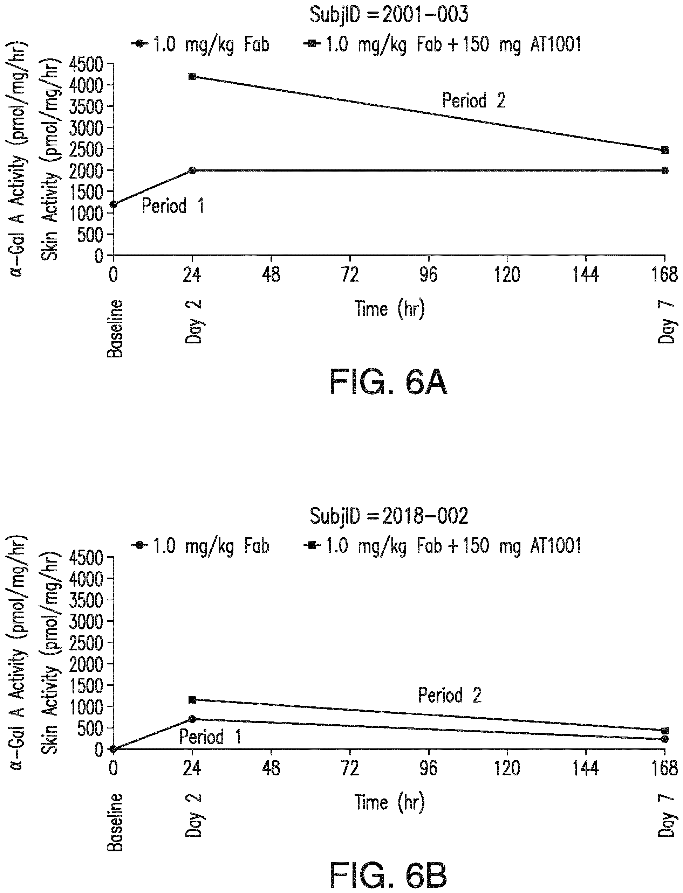

The increase in skin $\alpha$-Gal A activity following co-administration of 0.5 mg/kg or 1.0 mg/kg agalsidase beta and 150 mg migalastat are shown in Figures 4-6. Figure 5A shows the increase in skin $\alpha$-Gal A activity following co-administration of 0.5 mg/kg agalsidase beta and 150 mg migalastat in the patient who received a 40 min. longer ERT infusion during Period 2 than the other patients.

PBMC $\alpha$-Gal-A Activity Increases with Migalastat

For co-administration with migalastat (Period 2) relative to ERT alone (Period 1), the following mean increases in PBMC $\alpha$-Gal A activity were observed:

Mean increases for the 0.5 mg/kg agalsidase beta infusion:

- 2.3-, 2.0-, and 2.2-fold for 0.5 mg/kg agalsidase beta (N=4) on Days 2, 7, and 14, respectively

  ▪ Individual patient increase ranges: 1.4-3.1-, 1.4-2.3-, and 1.7-2.8-fold on Days 2, 7, and 14, respectively
  ▪ No change in skin activity from Period 1 on Day 7

Mean increases for the 1.0 mg/kg agalsidase beta infusion:

- 1.8-, 4.8- and 3.5-fold for 1.0 mg/kg agalsidase beta (N=2) on Days 2, 7 and 14, respectively
  ▪ Individual patient increases: 1.1- and 2.5-, 3.6- and 6.0-, and 1.7- and 5.4-fold on Days 2, 7, and 14, respectively

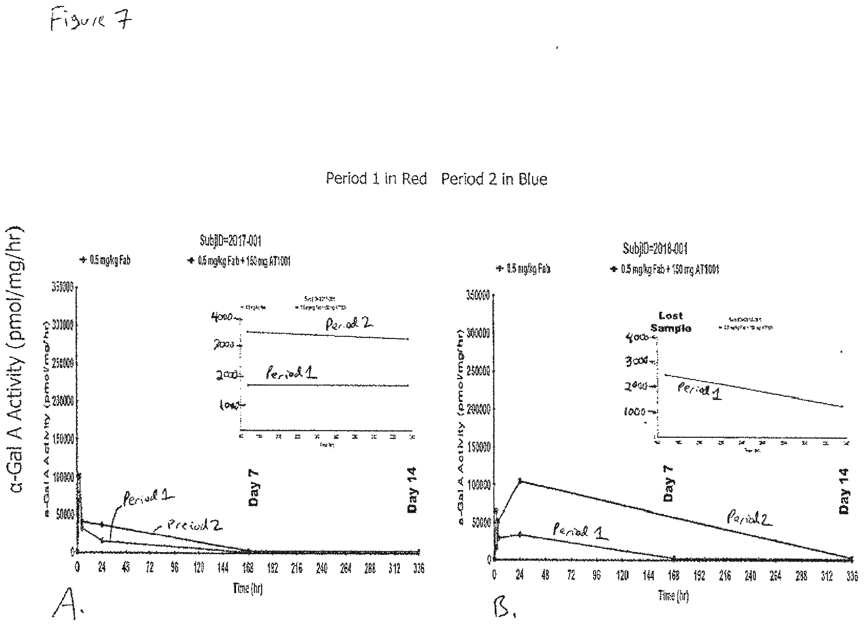

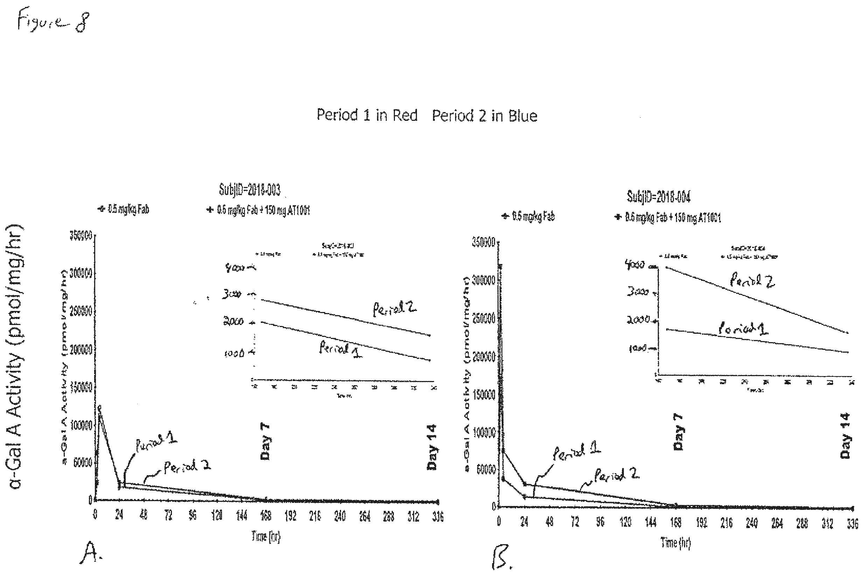

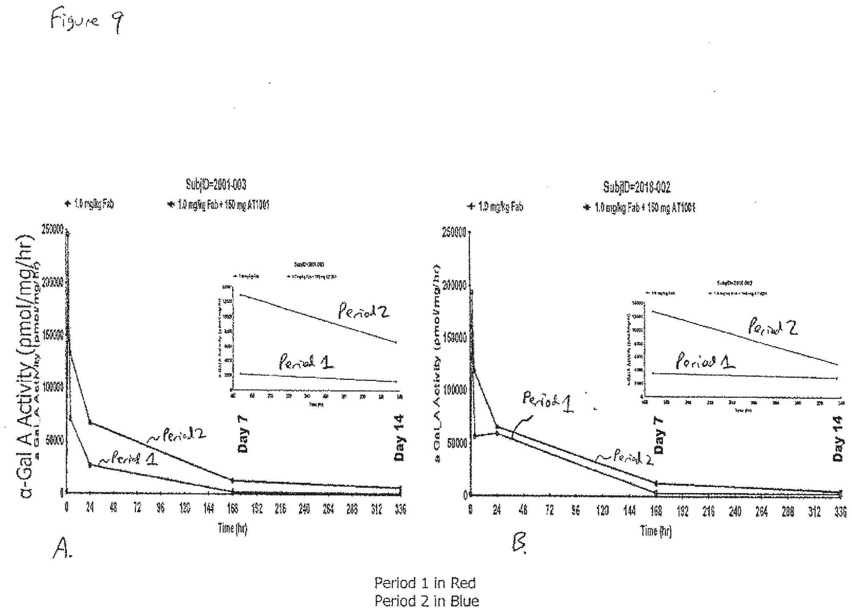

The increase in PBMC $\alpha$-Gal A activity following co-administration of 0.5 mg/kg or 1.0 mg/kg agalsidase beta and 150 mg migalastat are shown in Figures 7-9.

***Conclusion***:

150 mg migalastat interaction with 0.5 mg/kg and 1.0 mg/kg agalsidase beta resulted in $\alpha$-Gal A activity increases for:

All patients' plasma $\alpha$-Gal A AUC (N=6)
All patients' skin $\alpha$-Gal A on Day 2 (N=5), but only 3 of 5 patients on Day 7
All patients' PBMC $\alpha$-Gal A on Days 2, 7, and 14 (N=6)

150 mg migalastat interaction with 0.5 mg/kg or 1.0 mg/kg agalsidase beta resulted in 2-to 4-fold increases in $\alpha$-galactosidase A activity AUC, 1.1- to 3.9-fold increases in Day 2 skin $\alpha$-galactosidase A activity, and 1.1- to 6.0-fold increases in PBMC $\alpha$-galactosidase A activity for Days 2, 7, and 14 relative to agalsidase beta alone. On Day 7, four patients had increased $\alpha$-galactosidase A activity in skin following co-administration.

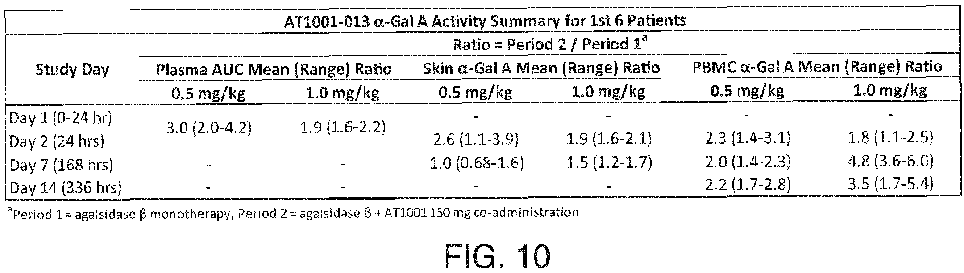

The 150 mg migalastat dose increased enzyme activity of the half-dose of agalsidase beta (0.5 mg/kg) better than the full dose (1.0 mg/kg) up to 24 hrs post dose in plasma, skin, and PBMC's; however the reverse was true (1.0 mg/kg > 0.5 mg/kg) at 7 and 14 days post dose in skin and PBMC's. A table summarizing the results is shown in Figure 10.

For agalsidase beta alone, all patients had increased PBMC $\alpha$-Gal A activity relative to baseline at all time points, however 2 patients had decreased skin $\alpha$-Gal A activity on Day 2 relative to baseline following the 0.5 mg/kg infusion.

**EXAMPLE 3: Dosing Regimen for the Treatment of Fabry Disease using Migalastat Hydrochloride and Agalsidase**

The following Example is an update of the study described in Example 2. The present example includes an additional subject for a total of seven subjects.

The objective of the present example is to evaluate the safety and PK of two doses of migalastat HCl (150 mg and 450 mg) co-administered with ERT (agalsidase) in males diagnosed with Fabry disease.

***Methods*** This is an ongoing, open-label, non-randomized, 2-stage, fixed-sequence study. Stage 1 is comprised of 3 periods.

- Period 1: IV infusion of ERT alone.
- Period 2: migalastat HCl (150 mg) orally administered 2 hours prior to IV infusion of the ERT (at the same dose as in period 1).

- Period 3: oral administration of migalastat HC1150 mg alone.

*Eligible patients*: Male, 18 to 65 years old with Fabry Disease.
*Inclusion criteria:*

- Body Mass Index (BMI) between 18-35.
- Initiated treatment with agalsidase at least 1 month before dosing.
- Estimated creatinine clearance (CLcr) $\geq$ 50 mL/min at screening.

Exclusion criteria:

- A documented transient ischemic attack, ischemic stroke, unstable myocardial infarction within 3 months before screening.
- Clinical significant unstable cardiac disease.
- Sensitivity to or concomitant therapy with iminosugars (e.g., miglustat, miglitol).

Subjects receive their current dose and regimen of agalsidase beta alone at one infusion (0.5 or 1.0 mg/kg for about 2 hrs) followed by oral migalastat HCl 150 mg administered two hours prior to agalsidase beta at their next infusion.
Five of the current seven subjects received 0.5 mg/kg every two weeks and two of the seven subjects received a dose of 1.0 mg/kg every four weeks.
In stage 2, a 450 mg dose of migalastat HCl will be studied.
Stages 1 and 2 will be repeated in unique subjects with an ERT infusion of agalsidase alpha (0.2 mg/kg for about 40 min).

*Samples*:

Serial blood samples were taken to 24 hours post dose for plasma $\alpha$-Gal A activity and protein levels each period. Blood samples for $\alpha$-Gal A activity in peripheral blood mononuclear cells (PBMCs) were taken at predose and on Days 1, 2, 7, and 14 of each period. Punch biopsies for skin $\alpha$-Gal A activity were taken at predose Period 1 and on Days 2 and 7 during Periods 1 and 2. Plasma $\alpha$-Gal A activity PK parameters include $C_{max}$, $T_{max}$, $AUC_{0-t}$, $AUC_{0-inf}$, and t½. Pharmacokinetic parameters are calculated using standard non-compartmental procedures (WINNONLIN version 5.0 or higher).
$\alpha$-Gal A activity in plasma skin, and PBMC lysates were measured by a fluorescence enzyme assay using 4-methylumbelliferyl-$\alpha$-D-galactopyranoside (4-MUG). $\alpha$-Gal A activity on 4 MUG was measured *in vitro* following serial dilutions to dissociate migalastat.
Western blot analysis of $\alpha$-Gal A protein were performed on plasma samples using anti-human $\alpha$-Gal A antibody. An rh$\alpha$-Gal A (agalsidase) standard curve was run to calculate the appropriate concentration of $\alpha$-Gal protein in each sample.
The safety parameters included adverse events (AEs), vital signs, clinical laboratory tests (hematology, serum chemistry, and urinalysis), electrocardiograms (ECGs), physical examinations, and use of concomitant medications.
*Results:* Preliminary results are available for Stage 1, Periods 1 and 2.
*Patient Disposition and Demographics*: Seven patients with plasma, skin and PBMC $\alpha$-Gal A activity from Stage 1, Periods 1 and 2 were evaluated.

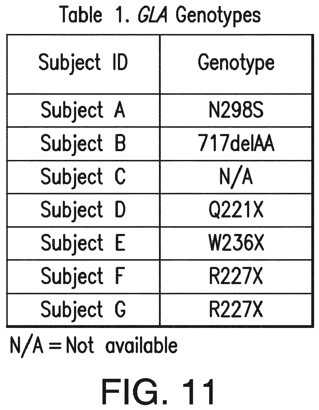

- All 7 patients received agalsidase beta alone during Period 1; all patients were co-administered 150 mg migalastat HCl 2 hours prior to initiation of agalsidase beta during Period 2.
- Two patients (identified as Subjects A and B) received IV infusions of agalsidase beta 1.0 mg/kg for 2-hr durations.
- Five patients (identified as Subjects C, D, E, F, and G) received IV infusions of agalsidase beta 0.5 mg/kg for 2-hr durations with 1 exception: Subject E was infused 2 hrs and 40 min during Period 2, but was infused for 2 hours during Period 1.
- All subjects were males with Fabry Disease aged 44-61 years, body mass index (BMI) ranged from 20.9-29.1 kg/m$^2$ and estimated CLcr ranged from 54-88 mL/min. The genotype for each subject is presented in Figure 11.

Safety:

To date, 12 adverse events (AEs) have been reported, one of which was serious. The serious AE was a transient ischemic attack (TIA) which occurred after the screening visit, but prior to dosing, was moderate in severity, required hospitalization, and was considered unrelated to study drug by the investigator. The TIA resolved without sequalae. All other AEs were mild in severity, all considered unrelated to study drug, and most resolved without treatment. Three AEs in three different subjects are ongoing: premature atrial contractions, atrial flutter, and lower extremity edema, all unrelated to study drug.

Plasma $\alpha$-Gal A Activity:

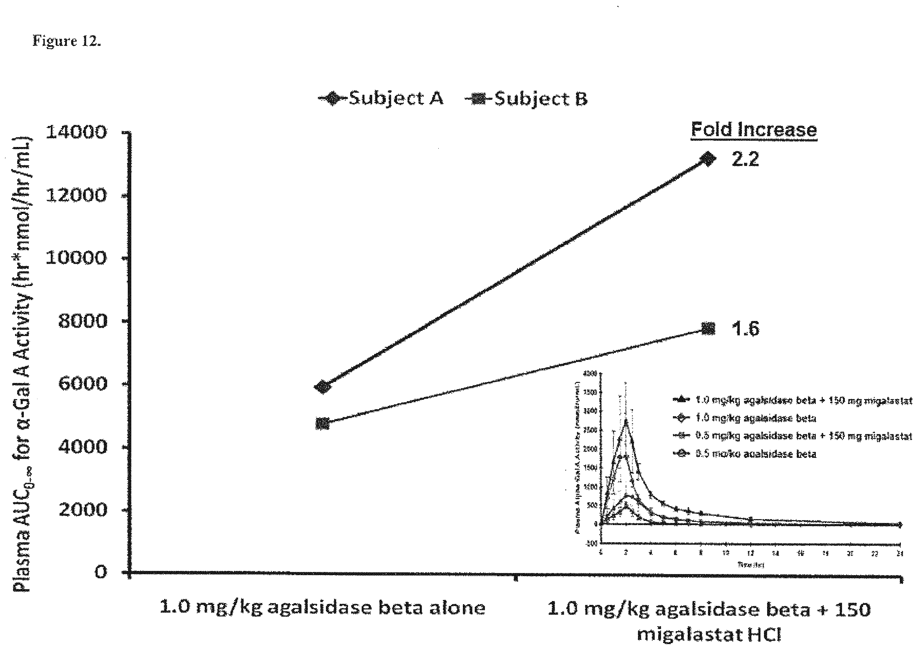

The plasma AUC (area under the curve) of $\alpha$-Gal A activity versus treatment with 1.0 mg/kg agalsidase beta alone, and treatment with 1.0 mg/kg agalsidase beta in combination with 150 mg migalastat HCl (with inserted means and standard deviations) activity-time profiles is shown in Figure 12 for subjects A and B. The combined treatment of 1.0 mg/kg agalsidase beta and 150 mg migalastat HCl resulted in 2.2 and 1.6 fold increases in $\alpha$-Gal A activity for subjects A and B, respectively, compared to treatment with 1.0 mg/kg agalsidase beta monotherapy.

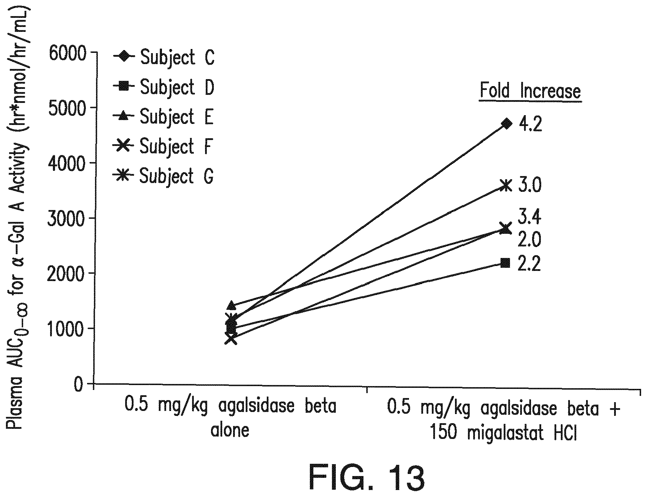

The plasma AUC (area under the curve) of $\alpha$-Gal A activity versus treatment with 0.5 mg/kg agalsidase beta alone, and treatment with 0.5 mg/kg agalsidase beta in combination with 150 mg migalastat HCl (with inserted means and standard deviations) activity-time profiles is shown in Figure 13 for subjects C-G. The combined treatment of 0.5 mg/kg agalsidase beta and 150 mg migalastat HCl resulted in a 2.0 to 4.2 fold increase in $\alpha$-Gal A activity compared to treatment with 0.5 mg/kg agalsidase beta monotherapy.

Plasma $\alpha$-Gal A activity increased at all time points for most subjects for co-administration relative to enzyme replacement therapy (ERT) alone with one exception. Subject E received an unbalanced infusion, 40 minutes longer during period 2 which caused relative decreased enzyme activity during the infusion phase. However, all Subject E time points post peak activity were increased relative to ERT alone. Additionally, consistent with Eng et al., Am. J. Hum. Genet. 68:711-722 (2001), exposures increased in a non-linear manner for $\alpha$-Gal A activity.

For co-administration with migalastat HCl (Period 2) relative to ERT alone (Period 1), all subjects had increased plasma $\alpha$-Gal A activity AUC as shown in Figures 12 and 13. The mean increase in plasma $\alpha$-Gal A activity AUC was 3.0-fold for 0.5 mg/kg agalsidase beta. Excluding the 2.0-fold patient with the unbalanced infusion duration, the mean increase was 3.2-fold. The mean increase in plasma $\alpha$-Gal A activity AUC was 1.9-fold for 1.0 mg/kg agalsidase beta.

Skin $\alpha$-Gal A activity

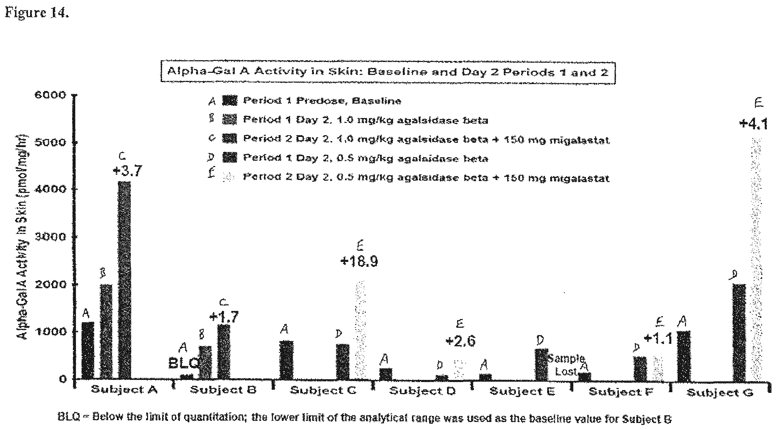

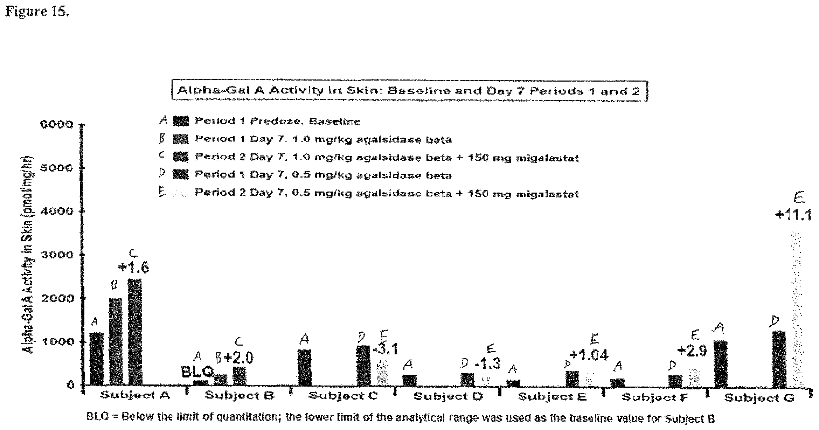

Figure 14 shows that treating subjects with 0.5 mg/kg or 1.0 mg/kg agalsidase beta in combination with 150 mg migalastat HCl increased $\alpha$-Gal A activity in Day 2 skin samples compared to enzyme monotherapy treatment alone. Figure 15 shows $\alpha$-Gal A activity in Day 7 skin samples following monotherapy and combined therapy with agalsidase beta and 150 mg migalastat HCl. The following mean increases in skin $\alpha$-Gal A activity were observed: 2.6-fold and 1.4-fold on Days 2 and 7, respectively, for 0.5 mg/kg agalsidase beta (N=5), and 1.9-and 1.5-fold on Days 2 and 7, respectively, for 1.0 mg/kg agalsidase beta (N=2).

PMBC $\alpha$-Gal A activity

The following mean increases in PBMC $\alpha$-Gal A activity were observed: 2.4-, 1.9-, and 2.1-fold on Days 2, 7, and 14, respectively, for 0.5 mg/kg agalsidase beta (N=5), and 1.8-, 4.8- and 3.5-fold for 1.0 mg/kg (N=2) agalsidase beta on Days 2, 7 and 14, respectively.

Western Blot analysis

No change was observed in plasma $\alpha$-Gal A protein by Western Blot analysis of Period 2 (co-administration) / Period 1 (ERT alone) AUC ratio for 5 out of 7 subjects. However, 2 subjects, (Subject C who received 0.5 mg/kg agalsidase beta and Subject G who receive 1.0 mg/kg agalsidase beta) had 20% increases in protein amount following co-administration relative to ERT alone (data not shown).

**Conclusion**

The interaction of 150 mg migalastat with 0.5 mg/kg and 1.0 mg/kg agalsidase beta resulted in $\alpha$-Gal A activity increases in plasma, skin, and PBMCs. Co-administration of 150 mg migalastat HCl with agalsidase beta was generally safe and well-tolerated.

The present application is not to be limited in scope by the specific embodiments described herein. Indeed, various modifications of the application in addition to those described herein will become apparent to those skilled in the art from the foregoing description and the accompanying figures. Such modifications are intended to fall within the scope of the appended claims.

It is further to be understood that all values are approximate, and are provided for description.

Patents, patent applications, publications, product descriptions, and protocols are cited throughout this application, the disclosures of which are incorporated herein by reference in their entireties for all purposes.

Paragraphs of advantage:

1. A method of treating Fabry disease in a subject, the method comprising administering from about 50 mg to about 600 mg of 1-deoxygalactonojirimycin and an effective amount of $\alpha$-Gal A enzyme replacement therapy to a patient in need thereof.

2. The method of 1, wherein the amount of 1-deoxygalactonojirimycin administered is about 150 mg to about 450 mg.

3. The method of 1, wherein the amount of 1-deoxygalactonojirimycin administered is selected from about 150 mg, about 300 mg and about 450 mg.

4. The method of 1, wherein the patient fasts for a period of time beginning about 0.5 to about 4 hours prior to and ending about 0.5 to about 4 hours following administration of 1-deoxygalactonojirimycin.

5. The method of 4, wherein the patient fasts for at least about 2 hours prior to and at least about 2 hours following administration of 1-deoxygalactonojirimycin.

6. The method of 1, wherein the 1-deoxygalactonojirimycin is administered simultaneously with to about 4 hours prior to the administration of the $\alpha$-Gal A enzyme replacement therapy.

7. The method of 6, wherein the 1-deoxygalactonojirimycin is administered about 2 hours prior to the administration of the $\alpha$-Gal A enzyme replacement therapy.

8. The method of 1, wherein the 1-deoxygalactonojirimycin is migalastat hydrochloride.

9. The method of 1, wherein the $\alpha$-Gal A enzyme replacement therapy is selected from agalsidase alfa and agalsidase beta.

10. The method of 1, wherein the 1-deoxygalactonojirimycin is administered as an adjuvant to the $\alpha$-Gal A enzyme replacement therapy.

11. The method of 1, wherein the 1-deoxygalactonojirimycin and $\alpha$-Gal A enzyme replacement therapy are administered as a combination therapy.

12. The method of 6, wherein a second dose of 1-deoxygalactonojirimycin is administered between the administration of the $\alpha$-Gal A enzyme replacement therapy and about 4 hours thereafter.

13. The method of 7, wherein the 1-deoxygalactonojirimycin and $\alpha$-Gal A enzyme replacement therapy are administered every 1 to 4 weeks.

14. The method of 13, wherein the 1-deoxygalactonojirimycin and $\alpha$-Gal A enzyme replacement therapy are administered every 2 weeks.

15. A kit for treating Fabry disease in a subject, the kit comprising from about 50 mg to about 600 mg of 1-deoxygalactonojirimycin and an effective amount of $\alpha$-Gal A enzyme replacement therapy.

16. The kit of 15, wherein the amount of 1-deoxygalactonojirimycin is selected from about 150 mg, about 300 mg and about 450 mg.

**Claims**

1. A pharmaceutical composition comprising migalastat hydrochloride, magnesium stearate and pregelatinized starch.

2. The pharmaceutical composition according to claim 1, wherein the migalastat hydrochloride, magnesium stearate and pregelatinized starch are formulated in a white, hard gelatin capsule.

3. The pharmaceutical composition according to claim 1, wherein the pharmaceutical composition is a solid dosage form comprises about 75-80% migalastat hydrochloride, about 0.1-2% magnesium stearate and about 20-25% pregelatinized starch.

4. The pharmaceutical composition according to claim 1, wherein the pharmaceutical composition is a capsule comprising about 76.5% migalastat hydrochloride, about 0.5% magnesium stearate and about 23% pregelatinized starch.

5. The pharmaceutical composition according to any one of the preceding claims for the treatment of Fabry disease.

6. The pharmaceutical composition according to any one of the preceding claims, wherein the pharmaceutical composition is administered as migalastat monotherapy.

7. The pharmaceutical composition according to any one of claims 1 to 5, wherein the pharmaceutical composition is co-administered with $\alpha$-Gal A enzyme replacement therapy.

8. The pharmaceutical composition according to claim 7, wherein the $\alpha$-Gal A enzyme replacement therapy comprises agalsidase alpha or agalsidase beta.

9. The pharmaceutical composition according to claim 7 or claim 8, wherein the pharmaceutical composition is orally administered about 2 hour prior to the administration $\alpha$-Gal A the enzyme replacement therapy.

10. The pharmaceutical composition according to any one of the preceding claims, wherein the pharmaceutical composition comprises 150 mg of migalastat hydrochloride.

Period = ERT Alone

-○- 2001-003, 1.0 mg/kg
-■- 2017-001, 0.5 mg/kg
-◆- 2018-001, 0.5 mg/kg
-▲- 2018-002, 1.0 mg/kg
-▼- 2018-003, 0.5 mg/kg
-●- 2018-004, 0.5 mg/kg

Period = Co-Administration

-○- 2001-003, 1.0 mg/kg+AT1001 150 mg
-■- 2017-001, 0.5 mg/kg+AT1001 150 mg
-◆- 2018-001, 0.5 mg/kg+AT1001 150 mg
-▲- 2018-002, 1.0 mg/kg+AT1001 150 mg
-▼- 2018-003, 0.5 mg/kg+AT1001 150 mg
-●- 2018-004, 0.5 mg/kg+AT1001 150 mg

FIG. 1

FIG. 2

| | Treatments: Period 1 = ERT Alone, Period 2 = Migalastat 150 mg + ERT Co-adminstration (ERT Duration of IV Infusion = 2 Hrs Except Where Noted*) | | | | | | | | | | | |
|---|---|---|---|---|---|---|---|---|---|---|---|---|
| | Partial AUC (hr*nmol/hr/mL) | | | | | | | | | | | |
| Time (hr) | Patient 2018-001 Dose 0.5 mg/kg | | Patient 2018-002 Dose 1.0 mg/kg | | Patient 2018-003 Dose 0.5 mg/kg | | Patient 2017-001 Dose 0.5 mg/kg | | Patient 2018-004 Dose 0.5 mg/kg | | Patient 2001-003 Dose 1.0 mg/kg | |
| | Period 1 | Period 2 | Period 1 | Period 2 | Period 1 | Period 2* | Period 1 | Period 2 | Period 1 | Period 2 | Period 1 | Period 2 |
| 0 | 0.00 | 0.00 | 0.00 | 0.00 | 0.00 | 0.00 | 0.00 | 0.00 | 0.00 | 0.00 | 0.00 | 0.00 |
| 0.5 | 2.99 | 31.10 | 74.56 | 133.50 | 12.22 | 1.03 | 28.66 | 70.38 | 45.40 | 112.52 | 276.71 | 283.12 |
| 1 | 18.97 | 143.08 | 379.78 | 534.54 | 68.51 | 24.55 | 126.36 | 297.15 | 166.88 | 369.60 | 928.19 | 1123.82 |
| 1.5 | 62.30 | 341.48 | 1001.95 | 1169.00 | 198.69 | 96.83 | 273.80 | 687.24 | 329.60 | 714.57 | 1803.68 | 2447.60 |
| 2 | 207.93 | 627.19 | 1849.25 | 2045.65 | 447.96 | 224.16 | 461.73 | 1239.04 | 521.60 | 1158.43 | 2749.94 | 4076.81 |
| 2.5 | 375.63 | 950.41 | 2593.54 | 2958.40 | 715.92 | 435.62 | 700.30 | 1853.76 | 677.62 | 1555.41 | 3487.41 | 5637.51 |
| 3 | 473.45 | 1195.50 | 3050.36 | 3676.31 | 887.24 | 775.73 | 894.77 | 2343.62 | 750.26 | 1795.89 | 3938.29 | 6722.99 |
| 4 | 596.72 | 1477.60 | 3582.33 | 4666.40 | 1087.89 | 1446.48 | 1055.83 | 2906.90 | 806.64 | 2068.24 | 4383.67 | 7937.54 |
| 5 | 656.14 | MS | 3892.62 | 5278.38 | 1191.81 | 1834.86 | 1099.04 | 3196.58 | 823.78 | 2236.38 | 4582.71 | 8676.49 |
| 6 | 703.93 | 1778.28 | 4090.01 | 5714.58 | 1253.43 | 2045.17 | 1118.23 | 3416.14 | 831.55 | 2368.00 | 4703.49 | 9204.47 |
| 7 | 743.73 | 1868.55 | 4230.26 | 6045.67 | 1298.82 | 2173.06 | 1127.20 | 3605.26 | 837.35 | 2469.07 | 4797.74 | 9645.30 |
| 8 | 775.60 | 1939.88 | 4332.47 | 6324.18 | 1328.99 | 2256.24 | 1131.47 | 3755.69 | 840.95 | 2553.10 | 4880.92 | 10033.03 |
| 12 | 854.08 | 2098.10 | 4564.01 | 7079.30 | 1386.69 | MS | 1139.51 | 4211.63 | 845.21 | 2777.56 | 5119.90 | 11110.71 |
| 24 | 968.26 | 2249.06 | 4759.31 | 7823.54 | 1444.71 | 2862.96 | 1146.05 | 4780.19 | 847.97 | 2912.86 | 5555.68 | 12647.97 |

MS = Missing Sample                    *Infusion duration = 2 hrs 40 min

# FIG. 3

FIG. 4A

FIG. 4B

Period 2: 40 min. longer ERT infusion; Day 2 skin sample lost

FIG. 5A

FIG. 5B

FIG. 6A

FIG. 6B

Figure 7

Figure 8

Period 1 in Red    Period 2 in Blue

Figure 9

Period 1 in Red
Period 2 in Blue

| AT1001-013 α-Gal A Activity Summary for 1st 6 Patients | | | | | | |
|---|---|---|---|---|---|---|
| | Ratio = Period 2 / Period 1[a] | | | | | |
| Study Day | Plasma AUC Mean (Range) Ratio | | Skin α-Gal A Mean (Range) Ratio | | PBMC α-Gal A Mean (Range) Ratio | |
| | 0.5 mg/kg | 1.0 mg/kg | 0.5 mg/kg | 1.0 mg/kg | 0.5 mg/kg | 1.0 mg/kg |
| Day 1 (0-24 hr) | 3.0 (2.0-4.2) | 1.9 (1.6-2.2) | - | - | - | - |
| Day 2 (24 hrs) | | | 2.6 (1.1-3.9) | 1.9 (1.6-2.1) | 2.3 (1.4-3.1) | 1.8 (1.1-2.5) |
| Day 7 (168 hrs) | - | - | 1.0 (0.68-1.6) | 1.5 (1.2-1.7) | 2.0 (1.4-2.3) | 4.8 (3.6-6.0) |
| Day 14 (336 hrs) | - | - | - | - | 2.2 (1.7-2.8) | 3.5 (1.7-5.4) |

[a]Period 1 = agalsidase β monotherapy, Period 2 = agalsidase β + AT1001 150 mg co-administration

# FIG. 10

Table 1. *GLA* Genotypes

| Subject ID | Genotype |
|---|---|
| Subject A | N298S |
| Subject B | 717delAA |
| Subject C | N/A |
| Subject D | Q221X |
| Subject E | W236X |
| Subject F | R227X |
| Subject G | R227X |

N/A = Not available

# FIG. 11

Figure 12.

FIG. 13

Figure 14.

Alpha-Gal A Activity in Skin: Baseline and Day 2 Periods 1 and 2

A ■ Period 1 Predose, Baseline
B ▨ Period 1 Day 2, 1.0 mg/kg agalsidase beta
C ■ Period 2 Day 2, 1.0 mg/kg agalsidase beta + 150 mg migalastat
D ■ Period 1 Day 2, 0.5 mg/kg agalsidase beta
E ▨ Period 2 Day 2, 0.5 mg/kg agalsidase beta + 150 mg migalastat

BLQ = Below the limit of quantitation; the lower limit of the analytical range was used as the baseline value for Subject B

Figure 15.

Alpha-Gal A Activity in Skin: Baseline and Day 7 Periods 1 and 2

A — Period 1 Predose, Baseline
B — Period 1 Day 7, 1.0 mg/kg agalsidase beta
C — Period 2 Day 7, 1.0 mg/kg agalsidase beta + 150 mg migalastat
D — Period 1 Day 7, 0.5 mg/kg agalsidase beta
E — Period 2 Day 7, 0.5 mg/kg agalsidase beta + 150 mg migalastat

BLQ = Below the limit of quantitation; the lower limit of the analytical range was used as the baseline value for Subject B

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/121826 A2 (AMICUS THERAPEUTICS INC [US]; PALLING DAVID [US]) 9 October 2008 (2008-10-09) | 1-10 | INV. A61K31/445 A61K38/47 |
| Y | * page 4, line 27 - line 28 * * page 13, line 27 - line 29 * * page 13, line 24 * | 1-10 | A61P3/00 A61K9/48 |
| Y | WO 99/62517 A1 (FAN JIAN QIANG [US]; ISHII SATOSHI [JP]) 9 December 1999 (1999-12-09) * claim 5 * | 1-10 | |
| Y | US 2006/153829 A1 (FAN JIAN-QIANG [US]) 13 July 2006 (2006-07-13) * example 3 * * claims 52-57 * | 1-10 | |
| Y | US 2010/266571 A1 (LOCKHART DAVID [US] ET AL) 21 October 2010 (2010-10-21) * claim 5 * | 1-10 | |
| A | US 2010/291060 A1 (STURK LISA MARIE [US] ET AL) 18 November 2010 (2010-11-18) | 1-10 | TECHNICAL FIELDS SEARCHED (IPC) A61K A61P |

The present search report has been drawn up for all claims

| Place of search | Date of completion of the search | Examiner |
|---|---|---|
| Munich | 1 July 2020 | Albayrak, Timur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 0164

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2020

| Patent document cited in search report | | Publication date | Patent family member(s) | | Publication date |
|---|---|---|---|---|---|
| WO 2008121826 | A2 | 09-10-2008 | AU | 2008232614 A1 | 09-10-2008 |
| | | | CA | 2682441 A1 | 09-10-2008 |
| | | | EP | 2142197 A2 | 13-01-2010 |
| | | | JP | 2010523578 A | 15-07-2010 |
| | | | US | 2010113517 A1 | 06-05-2010 |
| | | | WO | 2008121826 A2 | 09-10-2008 |
| WO 9962517 | A1 | 09-12-1999 | AT | 442851 T | 15-10-2009 |
| | | | AU | 4186199 A | 20-12-1999 |
| | | | CA | 2333965 A1 | 09-12-1999 |
| | | | CY | 1112845 T1 | 10-02-2016 |
| | | | CY | 1115583 T1 | 04-01-2017 |
| | | | DK | 1083899 T3 | 25-01-2010 |
| | | | DK | 2143420 T3 | 06-10-2014 |
| | | | EP | 1083899 A1 | 21-03-2001 |
| | | | EP | 2143420 A1 | 13-01-2010 |
| | | | ES | 2333561 T3 | 23-02-2010 |
| | | | ES | 2507515 T3 | 15-10-2014 |
| | | | JP | 4602548 B2 | 22-12-2010 |
| | | | JP | 5528941 B2 | 25-06-2014 |
| | | | JP | 2003528799 A | 30-09-2003 |
| | | | JP | 2010270146 A | 02-12-2010 |
| | | | PT | 1083899 E | 18-12-2009 |
| | | | PT | 2143420 E | 30-09-2014 |
| | | | US | 6274597 B1 | 14-08-2001 |
| | | | US | 6583158 B1 | 24-06-2003 |
| | | | US | 2002035072 A1 | 21-03-2002 |
| | | | US | 2002156100 A1 | 24-10-2002 |
| | | | US | 2002198225 A1 | 26-12-2002 |
| | | | US | 2003119874 A1 | 26-06-2003 |
| | | | US | 2004242539 A1 | 02-12-2004 |
| | | | US | 2005113415 A1 | 26-05-2005 |
| | | | US | 2006100241 A1 | 11-05-2006 |
| | | | US | 2007021381 A1 | 25-01-2007 |
| | | | US | 2010137229 A1 | 03-06-2010 |
| | | | US | 2011076765 A1 | 31-03-2011 |
| | | | US | 2014051725 A1 | 20-02-2014 |
| | | | US | 2014206718 A1 | 24-07-2014 |
| | | | US | 2015258129 A1 | 17-09-2015 |
| | | | US | 2016346262 A1 | 01-12-2016 |
| | | | WO | 9962517 A1 | 09-12-1999 |
| US 2006153829 | A1 | 13-07-2006 | BR | PI0407001 A | 10-01-2006 |
| | | | CA | 2514642 A1 | 19-08-2004 |
| | | | CA | 2814767 A1 | 19-08-2004 |
| | | | CA | 2814774 A1 | 19-08-2004 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 20 16 0164

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2020

| Patent document cited in search report | Publication date | Patent family member(s) | | Publication date |
|---|---|---|---|---|
| | | CN | 1756558 A | 05-04-2006 |
| | | CN | 102580066 A | 18-07-2012 |
| | | CN | 102580067 A | 18-07-2012 |
| | | CN | 102586205 A | 18-07-2012 |
| | | CY | 1116215 T1 | 08-02-2017 |
| | | CY | 1116463 T1 | 15-03-2017 |
| | | CY | 1116464 T1 | 15-03-2017 |
| | | CY | 1116747 T1 | 15-03-2017 |
| | | CY | 1118849 T1 | 10-01-2018 |
| | | DK | 1589993 T3 | 09-03-2015 |
| | | DK | 2332567 T3 | 13-07-2015 |
| | | DK | 2441467 T3 | 31-08-2015 |
| | | DK | 2444102 T3 | 13-07-2015 |
| | | DK | 2857036 T3 | 13-03-2017 |
| | | EP | 1589993 A2 | 02-11-2005 |
| | | EP | 2332567 A1 | 15-06-2011 |
| | | EP | 2441467 A1 | 18-04-2012 |
| | | EP | 2444102 A1 | 25-04-2012 |
| | | EP | 2857036 A1 | 08-04-2015 |
| | | EP | 3178486 A1 | 14-06-2017 |
| | | ES | 2531478 T3 | 16-03-2015 |
| | | ES | 2541785 T3 | 24-07-2015 |
| | | ES | 2541952 T3 | 28-07-2015 |
| | | ES | 2545619 T3 | 14-09-2015 |
| | | ES | 2619353 T3 | 26-06-2017 |
| | | ES | 2686775 T3 | 19-10-2018 |
| | | HK | 1158955 A1 | 18-09-2015 |
| | | HK | 1168778 A1 | 25-09-2015 |
| | | HK | 1169033 A1 | 24-12-2015 |
| | | HK | 1207291 A1 | 29-01-2016 |
| | | HU | E027113 T2 | 29-08-2016 |
| | | HU | E033381 T2 | 28-11-2017 |
| | | IL | 169562 A | 21-04-2016 |
| | | IL | 214440 A | 30-03-2017 |
| | | IL | 241315 A | 29-12-2016 |
| | | JP | 5589165 B2 | 17-09-2014 |
| | | JP | 5808675 B2 | 10-11-2015 |
| | | JP | 5875870 B2 | 02-03-2016 |
| | | JP | 6211573 B2 | 11-10-2017 |
| | | JP | 2006516645 A | 06-07-2006 |
| | | JP | 2011078413 A | 21-04-2011 |
| | | JP | 2012102128 A | 31-05-2012 |
| | | JP | 2012107020 A | 07-06-2012 |
| | | JP | 2016053071 A | 14-04-2016 |
| | | MX | 344587 B | 20-12-2016 |
| | | MX | PA05007822 A | 10-02-2006 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                        EP 20 16 0164

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2020

| Patent document cited in search report | Publication date | Patent family member(s) | | Publication date |
|---|---|---|---|---|
| | | PT | 1589993 E | 04-03-2015 |
| | | PT | 2332567 E | 23-09-2015 |
| | | PT | 2441467 E | 12-10-2015 |
| | | PT | 2444102 E | 17-09-2015 |
| | | PT | 2857036 T | 13-03-2017 |
| | | SI | 1589993 T1 | 29-05-2015 |
| | | SI | 2332567 T1 | 31-08-2015 |
| | | SI | 2441467 T1 | 30-10-2015 |
| | | SI | 2444102 T1 | 31-08-2015 |
| | | US | 2004180419 A1 | 16-09-2004 |
| | | US | 2006153829 A1 | 13-07-2006 |
| | | US | 2007178081 A1 | 02-08-2007 |
| | | US | 2018085437 A1 | 29-03-2018 |
| | | WO | 2004069190 A2 | 19-08-2004 |
| US 2010266571 A1 | 21-10-2010 | AU | 2008245578 A1 | 06-11-2008 |
| | | CA | 2685332 A1 | 06-11-2008 |
| | | EP | 2150254 A2 | 10-02-2010 |
| | | JP | 2010525084 A | 22-07-2010 |
| | | US | 2010266571 A1 | 21-10-2010 |
| | | US | 2015352093 A1 | 10-12-2015 |
| | | WO | 2008134628 A2 | 06-11-2008 |
| US 2010291060 A1 | 18-11-2010 | AR | 068144 A1 | 04-11-2009 |
| | | EP | 2185187 A1 | 19-05-2010 |
| | | US | 2010291060 A1 | 18-11-2010 |
| | | US | 2014037612 A1 | 06-02-2014 |
| | | US | 2015306187 A1 | 29-10-2015 |
| | | WO | 2009032171 A1 | 12-03-2009 |

EPO FORM P0459

For more details about this annex : see Official Journal of the European Patent Office, No. 12/82

page 3 of 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61451798
- US 61578201 B
- US 61596165 B

**Non-patent literature cited in the description**

- **FULLER et al.** *Clinical Chemistry,* 2005, vol. 51, 688-694
- **E.W. MARTIN.** Remington's Pharmaceutical Sciences
- **ENG et al.** *Am. J. Hum. Genet.,* 2001, vol. 68, 711-722